# EUROPEAN PATENT SPECIFICATION

(11) **EP 3 515 464 B1**
(45) Date of publication and mention of the grant of the patent: **05.11.2025**
(21) Application number: 17780585.0
(22) Date of filing: 21.09.2017
(51) Int. Cl.: A61K 35/74, A61P 11/02, A61P 37/08

(54) **AMMONIA OXIDIZING MICROORGANISMS FOR USE AND DELIVERY TO THE INTRANASAL SYSTEM**
AMMONIAKOXIDIERENDE MIKROORGANISMEN ZUR VERWENDUNG UND ABGABE AN DAS INTRANASALE SYSTEM
MICRO-ORGANISMES OXYDANT L'AMMONIAC DESTINÉS À UNE UTILISATION ET UNE ADMINISTRATION AU NIVEAU DU SYSTÈME INTRANASAL

(30) Priority: 21.09.2016 US 201662397642 P
(43) Date of publication of application: 31.07.2019
(73) Proprietor: Aobiome LLC, Cambridge, MA 02142 (US)
(72) Inventor: KRUEGER, Todd, Weston, MA 02493 (US); WHITLOCK, David R., Cambridge, MA 02140 (US)
(74) Representative: Mathys & Squire
(86) International application number: PCT/US2017/052671
(87) International publication number: WO 2018/057710

(56) References cited:
- WO-A1-2015/179664
- WO-A1-2015/179669
- WO-A1-2016/161285
- WO-A1-2017/004557
- WO-A2-2015/160911
- ANONYMOUS: "Ao + Mist Product Description", MINTEL DATABASE, 1 March 2016 (2016-03-01), XP055427567, Retrieved from the Internet <URL:http://www.gnpd.com/sinatra/recordpage/3882415/from_search/MkpL720iNM/?page=1> [retrieved on 20171122]
- "The Hygiene Hypothesis and Darwinian Medicine", 1 January 2009, BIRKHÄUSER BASEL, Basel, ISBN: 978-3-7643-8903-1, article DAVID R. WHITLOCK ET AL: "Soil bacteria, nitrite and the skin", pages: 103 - 115, XP055427427, DOI: 10.1007/978-3-7643-8903-1_6

## Description

### Cross-Reference to Related Applications

This application claims priority under 35 U.S.C. § 119(e) to U.S. Provisional Patent Application Serial No. 62/397,642 as filed on September 21, 2016.

### Field of the Technology

Aspects relates generally to the microbiome and, more specifically, to the restoration of ammonia oxidizing microorganisms in relation to the microbiome.

### Background

Bacteria and other microorganisms are ubiquitous in the environment. The discovery of pathogenic bacteria and the germ theory of disease have had a tremendous effect on health and disease states. Microorganisms are a normal part of the environment of all living things and may be beneficial. In the nasal passages, inhaled air passes over specialized nasal structures. Microorganisms, ambient molecules, and particles elicit responses in the nasal passages and become trapped in a layer of high viscosity mucus. The internal nasal surface is characterized by groups of ciliated cells which act to transport the layer of high viscosity mucus for local or systemic delivery.

WO 2015/160911, WO 2015/179664 and WO 2015/179669 mention compositions comprising ammonia-oxidizing bacteria.

### Summary

The invention is defined according to the claims. Thus, the invention provides a preparation comprising ammonia oxidizing microorganisms (AOM) for use in a method of treating allergic rhinitis in a subject, the method comprising: administering an effective amount of the preparation comprising AOM to the nasal cavity of the subject.

In one embodiment, intranasal administration comprises topical application, inhalation, instillation, or olfactory transfer administration.

In one embodiment, (i) the nasal cavity of the subject is substantially cleared when the preparation is administered; or (ii) the preparation is to be administered subsequent to administration of an antibiotic or a nasal cavity cleansing preparation.

In one embodiment, (i) the method further comprises administering a second amount of the preparation to the subject; (ii) the therapeutically effective dose of AOM is about or greater than about 1 x 10³, 10⁴, 10⁵, 10⁶, 10⁷, 10⁸, 10⁹, 10¹⁰, 10¹¹, 10¹², 10¹³, or 10¹⁴ CFU; (iii) the preparation comprises AOM in a buffer solution, e.g., an aqueous buffer solution, optionally comprising disodium phosphate and magnesium chloride, for example, 50 mM Na₂HPO₄ and 2 mM MgCl₂ in water; or (iv) a biome-friendly product is used in connection with the preparation comprising AOM.

In one embodiment, the preparation: (i) is formulated to be compatible with the mucous membrane of the nasal cavity of the subject; (ii) is formulated for immediate release or extended release; (iii) is to be administered as part of a combination therapy; or (iv) is substantially free of other organisms; or (v) comprises between about 1 x 10³ CFU/mL to about 1 x 10¹⁴ CFU/mL AOM.

In one embodiment, the AOM: (i) comprise ammonia oxidizing bacteria (AOB); (ii) comprise *Nitrosomonas, Nitrosococcus, Nitrosospira, Nitrosocystis, Nitrosolobus, Nitrosovibrio,* and combinations thereof; (iii) is *Nitrosomonas eutropha* (*N. eutropha*); (iv) is *N. eutropha* D23, having ATCC accession number PTA-121157; or (v) are capable of converting ammonia or ammonium to nitrite at a rate of at least about 1 pmol/min/mg protein, e.g., at least about 0.1 nmol/min/mg protein.

In one embodiment, the preparation: (i) is a nasal drop, spray, aerosol, or mist; (ii) is formulated for treatment of a neurological disorder in a subject; (iii) is formulated for treatment of a nasal or sinus disorder in a subject; or (iv) comprises AOM and other organisms, e.g., a community of organisms.

A method of delivering AOM to a nasal cavity of a subject is disclosed. The method may comprise administering an effective amount of a
preparation comprising AOM to the nasal cavity of the subject, wherein the AOM colonize a target tissue of the nasal cavity.

A method of treating a neurogenic inflammation in a subject is also disclosed. The neurogenic inflammation may comprise, for example, oxidative stress. The method may comprise administering to the subject an effective amount of a preparation comprising AOM, thereby treating the neurogenic inflammation in the subject.

A method of treating a neurological disorder in a subject is also disclosed. The method may comprise administering to the subject an effective amount of a preparation comprising AOM, thereby treating the neurological disorder in the subject.

A method of treating a nasal or sinus disorder in a subject is also disclosed. The method may comprise administering to the subject an effective amount of a preparation comprising AOM, thereby treating the nasal or sinus disorder in the subject.

A method of treating a systemic inflammatory condition in a subject is also disclosed. The method may comprise intranasally administering to the subject an effective amount of a preparation comprising AOM, thereby treating the systemic inflammatory condition in the subject. The systemic inflammatory condition may be associated with one or more of: headaches (e.g., migraines), cardiovascular diseases (e.g., hypertension), inflammation, immune responses, autoimmune disorders, liver diseases, infections, neurological diseases, psychiatric disorders, nitric oxide disorders, urea cycle disorders, congestion, vasodilation disorders, skin diseases, wound healing, reactions to insect bites, ophthalmic disorders, connective tissue disorders, and certain viral, bacterial, or fungal infections.

A method of treating a headache or a migraine headache in a subject is also disclosed. The method may comprise intranasally administering to the subject an effective amount of a preparation comprising AOM, thereby treating the headache or migraine headache in the subject.

An amount and/or a frequency of administration may be sufficient to increase mucus thickness in at least a portion of the nasal cavity of the subject. Administering the preparation may result in anti-triggering of ischemic pre-conditioning or modulation of an ATP
level in the subject. An amount and/or a frequency of administration may be sufficient to induce ischemic anti-triggering in the subject.

The preparation comprising AOM may be administered intranasally to a nasal cavity of a subject. The subject may have a substantially cleared nasal cavity when the preparation is administered. The preparation may be administered subsequent to administration of an antibiotic or a nasal cavity cleansing preparation. A target percentage of administered AOM may be transferred to a central nervous system (CNS) of the subject. The method may further comprise administering water or a buffer solution, e.g., an aqueous buffer solution to the subject subsequent to administering the preparation.

Administering the preparation may result in decongestion, decreased sinus pressure, or modulation of an inflammatory response. The neurological disorder may be an inflammatory condition. The inflammatory condition may be a nasal or sinus disorder. The nasal or sinus disorder may be allergic rhinitis. **In** the claimed invention, the preparation is for use in a method of treating allergic rhinitis in a subject.

The inflammatory condition may be neurogenic inflammation, e.g., associated with headache, neuropathy (e.g., diabetic neuropathy, peripheral neuropathy, Lewy body neuropathy), epilepsy, systemic sclerosis, multiple sclerosis, amyotrophic lateral sclerosis, Alzheimer's Disease, Parkinson's Disease, white matter hyperintensities, diabetic retinopathy, anxiety, post-traumatic stress disorder, chronic fatigue syndrome, fibromyalgia, depression, insomnia, arthritis, rheumatoid arthritis, allergic rhinitis, dilative cardiomyopathy, atherosclerosis, cardioprotection, heart failure, hypertension (e.g., pulmonary hypertension, gestational hypertension, portal hypertension), eclampsia, pre-eclampsia, capillary rarefaction, peripheral vasculopathy, gestational diabetes, type 2 diabetes, obesity, metabolic syndrome, kidney failure, liver failure, pancreatitis, or hepatitis.

The nasal or sinus disorder may relate to an allergen, a bacterial infection, or a viral infection, e.g., the coronavirus, rhinovirus, meningitis, or influenza. The inflammatory condition may be associated with an injury, cancer, or an infection.

Administration may precede or follow a medical procedure, e.g., a catheterization, endoscopy, intubation, e.g., nasogastric intubation, administration of a nasal cannula, or a dental procedure. The inflammatory condition may be associated with or follow an injury, e.g., spinal cord injury, head trauma, or brain injury.

The nasal or sinus disorder may be characterized by congestion or sinus pressure. The neurological disorder may be a degenerative disorder or a genetic disorder. The neurological disorder may comprise a psychological disorder.

Administration may be to a deposit tissue or directly to a target tissue. A deposit tissue, target tissue, or both may be a mucous membrane of the subject. The deposit tissue, target tissue, or both may be associated with a nasal cavity of the subject. The deposit tissue, target tissue, or both may be a nasal cavity, septal wall, nasal valve, nostril, nasopharanyx, vestibular area, turbinate (e.g., inferior, middle, superior), meatus (e.g., inferior, middle, superior), concha (e.g., inferior, middle, superior), maxillary sinus, sphenoidal sinus, sphenoethmoidal recess, ethmoidal bulla, semi-lunar hiatus, nasolacrimal duct, frontonasal duct, or olfactory region of the subject.

The target tissue may be associated with a desired local effect. The target tissue may be associated with a desired systemic effect. For example, a desired systemic effect may involve treatment of one or more of: headaches, cardiovascular diseases, inflammation, immune responses, autoimmune disorders, liver diseases, infections, neurological diseases, psychiatric disorders, nitric oxide disorders, urea cycle disorders, congestion, vasodilation disorders, skin diseases, wound healing, reactions to insect bites, ophthalmic disorders, connective tissue disorders, and certain viral, bacterial, or fungal infections.

Administering an effective amount of the preparation may promote endothelial function. Administering an effective amount of the preparation may change or alter a level of nitrite or NO at a target tissue or in circulation. Administering an effective amount of the preparation may modulate a microbiome associated with the intranasal system of the subject. Administering an effective amount of the preparation may modulate a microbiome associated with the CNS of the subject. Administering an effective amount of the preparation may modulate a systemic microbiome associated with a remote system, e.g., gastrointestinal system, circulatory system, respiratory system, endocrine system, or immune system, of the subject.

Administration may be device-assisted. The preparation may be administered prior to onset, during incidence, or subsequent to the subsiding of a gastrointestinal condition. The preparation may be administered in response to an inflammatory symptom, trigger or warning sign, e.g. discomfort, a change in sinus pressure, or a stress state. A method
may involve determining if the subject is in need of treatment for a neurological disorder. A method may involve determining if the subject is in need of treatment for a nasal or sinus disorder.

The preparation may be administered as a solution, suspension, emulsion, ointment, bougie, powder, gel, hydrogel, or liquid, e.g. drop, spray, aerosol, or mist. The preparation may be formulated as a drop, spray, aerosol, or mist. The preparation may include microspheres or microcapsules. The preparation may be formulated to be compatible with the mucous membrane of the nasal cavity of the subject. The preparation may be formulated for immediate release or extended release. The preparation may be formulated to deliver nitrite or NO to a target tissue, locally or systemically. The preparation may be formulated for transmucosal delivery and/or circulation, *e.g*. locally or systemically.

Administration may reduce inflammation, congestion, sinusitis, asthma, sneezing, sinus pressure, or discomfort in the subject.

A treatment method may further comprise administering a second amount of the preparation to the subject. The preparation may be administered as part of a combination therapy. The method may further comprise administering a second treatment in combination with the preparation. The preparation may be administered for a period of time prior to initiating the second treatment, concurrently with the second treatment, or for a period of time subsequent to ceasing the second treatment. The second treatment may be administered via an alternate mode of administration, e.g. via inhalation or enteral technique. The subject may have a therapeutic level of a second treatment. The preparation may be administered in conjunction with an anti-inflammatory agent. The preparation may be administered in conjunction with a medical approach that treats, e.g., is approved to treat or is commonly used to treat, the relevant disease or disorder, or a symptom of the relevant disease or disorder. The preparation may be administered before or after a surgical or diagnostic procedure. The second treatment may comprise a surgical procedure. The preparation may be administered in conjunction with decongestant, probiotic, therapeutic, exercise, or stress management.

The preparation may be administered in combination with a therapeutic treatment for headache, neuropathy (e.g., diabetic neuropathy, peripheral neuropathy,
Lewy body neuropathy), epilepsy, systemic sclerosis, multiple sclerosis, amyotrophic lateral sclerosis, Alzheimer's Disease, Parkinson's Disease, white matter hyperintensities, diabetic retinopathy, anxiety, post-traumatic stress disorder, chronic fatigue syndrome, fibromyalgia, depression, insomnia, arthritis, rheumatoid arthritis, allergic rhinitis, dilative cardiomyopathy, atherosclerosis, cardioprotection, heart failure, hypertension (e.g., pulmonary hypertension, gestational hypertension, portal hypertension), eclampsia, pre-eclampsia, capillary rarefaction, peripheral vasculopathy, gestational diabetes, type 2 diabetes, obesity, metabolic syndrome, kidney failure, liver failure, pancreatitis, or hepatitis. The preparation may be administered in conjunction with nitrite, nitrate, and/or NO.

The effective amount may be a therapeutically effective dose of AOM. The therapeutically effective dose of AOM is about or greater than about 1 x 10³, 10⁴, 10⁵, 10⁶, 10⁷, 10⁸, 10⁹, 10¹⁰, 10¹¹, 10¹², 10¹³, or 10¹⁴ CFU. The preparation may be administered as an analgesic and/or as a prophylactic. The preparation may be self-administered. The preparation may be administered about 1, 2, 3, 4, 5, 6, 7, 8, 9, 10, 11, 12, 13, 14, 15, 16, 17, 18, 19, 20, 21, 22, 23, or 24 times per day. The preparation may be administered for about 1-3, 3-5, 5-7, 7-9, 5-10, 10-14, 12-18, 12-21, 21-28, 28-35, 35-42, 42-49, 49-56, 46-63, 63-70, 70-77, 77-84, or 84-91 days. The preparation may be administered within 30, 60, 90, 120, 150, or 180 minutes of the subject waking from sleep. The preparation may be administered within 30, 60, 90, 120, 150, or 180 minutes prior to the subject sleeping. The preparation may be administered within 30, 60, 90, 120, 150, or 180 minutes of the subject eating. The preparation may be administered 30, 60, 90, 120, 150, or 180 minutes before the subject cleanses or showers.

The subject may be female. The subject may be male. The subject may be characterized as one of the following ethnicity/race: Asian, black or African American, Hispanic or Latino, white, or multi-racial. The subject may have a disrupted microbiome. The subject may be of an age less than 1, or between 1-5, 5-10, 10-20, 20-30, 30-40, 40-50, 50-60, or over 60 years.

The preparation may comprise AOM in a buffer solution, e.g., an aqueous buffer solution. The buffer solution, e.g., aqueous buffer solution, comprises disodium phosphate and magnesium chloride, for example, 50 mM Na₂HPO₄ and 2 mM MgCl₂ in water. The buffer solution e.g., aqueous buffer solution, consisting essentially of disodium phosphate and magnesium chloride, for example, 50 mM Na₂HPO₄ and 2 mM MgCl₂ in water. The buffer solution, e.g., aqueous buffer solution, consists of disodium phosphate and magnesium chloride, for example, 50 mM Na₂HPO₄ and 2 mM MgCl₂ in water. The preparation may be characterized by a physiological pH level. The preparation may further comprise or be administered concurrently with a compound that promotes growth or metabolism of the AOM, NO production, and/or urease activity. The preparation may comprise at least one of ammonia, ammonium salts, and urea. The preparation may comprise a controlled release material, e.g., slow release material. The preparation may further comprise an excipient, e.g., a pharmaceutically acceptable excipient. The excipient may comprise an absorption or penetration enhancer, preservative, antioxidant, buffer, chelating agent, ion exchange agent, solubilizing agent, suspending agent, thickener, surfactant, wetting agent, tonicity-adjusting agent, enzyme inhibitor, or vehicle for proper drug delivery. The preparation may comprise a mucoadhesive agent, disintegrant, chelator, coating agent, modified-release product, or filler. The preparation may be substantially free of other organisms.

The preparation may comprise between about 1 x 10³ CFU/mL to about 1 x 10¹⁴ CFU/mL AOM. The preparation may comprise between about 1 x 10⁹ CFU/mL to about 10 x 10⁹ CFU/mL AOM. The AOM may comprise ammonia oxidizing bacteria (AOB). The AOM may consist essentially of AOB. The AOM may consist of AOB. The AOM may comprise *Nitrosomonas, Nitrosococcus, Nitrosospira, Nitrosocystis, Nitrosolobus, Nitrosovibrio,* and combinations thereof. The AOM may be *Nitrosomonas eutropha* (*N. eutropha*)*.* The AOM may be *N. eutropha* D23, having ATCC accession number PTA-121157. The AOM may comprise ammonia oxidizing archaea (AOA). The AOM may be capable of converting ammonia or ammonium to nitrite at a rate of at least about 1 pmol/min/mg protein. The AOM may be capable of converting ammonia or ammonium to nitrite at a rate of at least about 0.1 nmol/min/mg protein.

The treatment may comprise a surgical procedure. The excipient may comprise an anti-adherent, binder, coat, disintegrant, filler, flavor, color, lubricant, glidant, sorbent preservative, or sweetener.

A biome-friendly product may be used in connection with the administered preparation comprising AOM.

A preparation comprising AOM, as disclosed herein, may be for intranasal administration to a subject.

The preparation may be a nasal drop, spray, aerosol, or mist.

A preparation may comprise AOM. The preparation may be for treatment of a neurological disorder in a subject. The preparation may be for treatment of a nasal or sinus disorder in a subject.

The preparation may be packaged for single use. The preparation may be packaged for multiple use.

The preparation may further comprise other organisms, e.g., a community of organisms.

A device may be configured to administer a preparation comprising AOM to a target tissue of a nasal cavity of a subject.

A kit may comprise a preparation comprising AOM, e.g., for delivery to an intranasal system of a subject or for treatment of a neurological disorder or nasal or sinus disorder in a subject.

### Detailed Description

The present disclosure provides for various methods or modes of introducing ammonia oxidizing microorganisms to a subject. These methods or modes comprise administering to a subject ammonia oxidizing microorganisms, for example, a preparation, composition, formulation, or product comprising ammonia oxidizing microorganisms. Ammonia oxidizing microorganisms may
therefore generally be restored to a microbiome of the subject. Ammonia oxidizing microorganisms may comprise or consist essentially of live ammonia oxidizing microorganisms.

Preparations, compositions, and/or formulations, e.g., including cosmetic products, therapeutic products, consumer products, non-natural products, natural products, and fortified natural products, comprising, consisting essentially of, or consisting of ammonia oxidizing microorganisms are disclosed. These preparations, compositions, and/or formulations are disclosed herein for use in various applications, e.g., cosmetic and/or therapeutic applications. The preparations, compositions, and/or formulations may be administered in an effective amount for an intended use, e.g., a cosmetic or a therapeutic application. Preparations, compositions, and/or formulations comprising ammonia oxidizing microorganisms for various modes of administration to a subject are provided. Preparations, compositions, and/or formulations comprising ammonia oxidizing microorganisms for use in the treatment of various conditions and/or disorders in a subject are provided. Methods of treating a subject for various conditions and/or disorders via administration of ammonia oxidizing microorganisms are disclosed. Devices for use in administering ammonia oxidizing microorganisms to a subject are also provided.

### Microbiology

Essentially any ammonia oxidizing microorganism (AOM) can be used or implemented. The ammonia oxidizing microorganisms may generally be autotrophic. The ammonia oxidizing microorganisms may generate nitrite and/or nitric oxide from ammonia.

Properties of autotrophic ammonia oxidizing bacteria (AOB), for example, are well described by Whitlock in U.S. Patent No. 7,820,420. Since that filing, the class of autotrophic microorganisms that oxidize ammonia for ATP production has been expanded to encompass ammonia oxidizing archaea (AOA), and archaea have been moved out of the class of bacteria and into their own distinct class. For the purposes of this disclosure, any and all autotrophic ammonia oxidizing microorganisms that share the properties of oxidation of ammonia to generate ATP can be implemented. AOM, including both AOB and AOA, share the necessary properties of oxidation of ammonia into NO and nitrite and all known AOM lack capacity for virulence because of their inability to use organic substrates for ATP generation. Bacteria can utilize ammonia at higher concentrations, while archaea can utilize ammonia at lower concentrations. Physiological levels of ammonia are within the range that both bacteria (AOB) and archaea (AOA) can utilize. Any reference specifically to ammonia oxidizing bacteria throughout this disclosure should be considered equally applicable to any ammonia oxidizing microorganism, e.g., any ammonia oxidizing archaea, and these terms may all be used interchangeably herein.

Ammonia oxidizing bacteria (AOB) are ubiquitous Gram-negative obligate bacteria with a unique capacity to generate energy exclusively from the conversion of ammonia to nitrite. Ammonia oxidizing bacteria (AOB) of the genus *Nitrosomonas* may be Gram-negative obligate autotrophic (chemolithoautotrophic) bacteria with a unique capacity to generate nitrite and nitric oxide exclusively from ammonia as an energy source. They are widely present both in soil and water environments and are essential components of environmental nitrification processes. These bacteria have beneficial properties, e.g., in connection with various cosmetic and therapeutic uses. Without wishing to be bound to any particular theory, due to the roles of nitrite and nitric oxide as important components of several physiological functions, such as vasodilation, inflammation and wound healing, these bacteria may have various beneficial properties for both healthy and immunopathological conditions. These bacteria are safe for use in humans because they are slow-growing, cannot grow on organic carbon sources, may be sensitive to soaps and antibiotics, and have never been associated with any disease or infection in animals or humans.

Ammonia oxidizing microorganisms generate coenzyme Q 8 (CoQ8) as a byproduct of the process by which they generate nitrite and nitric oxide. CoQ8 is a coenzyme Q having 8 carbons in its isoprenoid side chain. Without wishing to be bound to any particular theory, due to the role of coenzyme Q as an important component of several cell functions, such as mediating cell signaling and preventing cell death (anti-aging), these microorganisms' beneficial properties may further be enhanced by their specific ability to generate CoQ8.

Ammonia oxidizing bacteria may catalyze the following reactions.

At a neutral pH level, ammonia generated from ammonium around neutral pH conditions is the substrate of the initial reaction. The conversion of ammonia to nitrite takes place in two steps catalyzed respectively by ammonia monooxygenase (AMO) and hydroxylamine oxidoreductase (HAO), as follows:

NH₃ + 2H⁺ + 2e- + O₂ → NH₂OH + H₂O (A)

NH₂OH + H₂O → NO₂⁻ + 4e- + 5H⁺ (B)

In some instances, reaction B is reported as follows, to indicate nitrous acid (HNO₂) formation at low pH:

NH₂OH + H₂O → HNO₂ + 4e- + 4H⁺

NH₄⁺ and NH₃ may be used interchangeably throughout the disclosure.

Examples of ammonia oxidizing bacteria include *Nitrosomonas eutropha* strains, *e.g.,* D23 and C91 as discussed herein, and other bacteria in the genera *Nitrosomonas, Nitrosococcus, Nitrosospira, Nitrosocystis, Nitrosolobus,* and *Nitrosovibrio.* D23 *Nitrosomonas eutropha* strain refers to the strain, designated AOB D23-100, deposited with the American Tissue Culture Collection (ATCC) (10801 University Blvd., Manassas, VA, USA) on April 8, 2014 having accession number PTA-121157. "AOB D23-100" may also be referred to as D23 or B244 throughout this disclosure.

Examples of ammonia oxidizing archaea include archaea in the genera *Methanobrevibacter, Methanosphaera, Methanosarcina, Nitroscaldus, Nitrosopumilus,* and *Nitrososphaera* (e.g. *Nitrososphaera viennensis, Nitrososphaera gargensis*)*.* Different phylotypes of archaea, e.g., methanogens and halphilic archaeon, may be included in the preparations disclosed herein. Examples of archaea further include archaea in the lineages of phyla Euryarchaeota (e.g. *Methanosarcina*), Crenarchaeota, Aigarchaeota, and Thaumarchaeota (e.g. *Giganthauma karukerense, Giganthauma insulaporcus, Caldiarchaeum subterraneum, Cenarchaeum symbiosum).*
The ammonia oxidizing microorganism may be a strain described in International (PCT) Patent Application Publication No. WO2015/160911 (International (PCT) Patent Application Serial No. PCT/US2015/025909 as filed on April 15, 2015).

Ammonia oxidizing microorganisms may exist in several metabolic states, e.g. growth state, storage state, and/or polyphosphate loading state.

Ammonia oxidizing microorganisms may have desirable properties, e.g., optimized properties, such as the ability to suppress growth of pathogenic bacteria, and an enhanced ability to produce nitric oxide and nitric oxide precursors.

Optimized *Nitrosomonas eutropha (N. eutropha),* as that term is used herein, refers to an *N. eutropha* having an optimized growth rate; an optimized NH₄⁺ oxidation rate; and/or optimized resistance to NH₄⁺. *N. eutropha* described herein may differ from naturally occurring *N. eutropha* by at least one nucleotide, *e.g.,* a nucleotide in a gene selected from ammonia monooxygenase, hydroxylamine oxidoreductase, cytochrome c554, and cytochrome c_{M}552. The difference can arise, *e.g*., through selection of spontaneously arising mutation, induced mutation, or directed genetic engineering, of the *N. eutropha. N. eutropha* described herein may differ from a naturally occurring *N. eutropha* in that it has a constellation of alleles, not present together in nature. These differences may provide for one or more of a treatment or prevention of a disease or condition, such as but not limited to one associated with low nitrite levels.

Any ammonia oxidizing bacteria, e.g., *N. eutropha,* for example *N. eutropha* referred to as "D23", also known as "B244" or "AOB D23-100" may have several of the above-described properties. Any ammonia oxidizing archaea (AOA) may also have several of the above-described properties.

The AOBs contemplated in this disclosure may comprise mutations relative to wild-type AOBs. These mutations may, *e.g*., occur spontaneously, be introduced by random mutagenesis, or be introduced by targeted mutagenesis. For instance, the AOBs may lack one or more genes or regulatory DNA sequences that wild-type AOBs typically comprise. The AOBs may also comprise point mutations, substitutions, insertions, deletions, and/or rearrangements relative to the sequenced strain or a wild-type strain. The AOBs may be a purified preparation of optimized AOBs.

The AOBs may be transgenic. For instance, it may comprise one or more genes or regulatory DNA sequences that wild-type ammonia oxidizing bacteria lacks. More particularly, the ammonia oxidizing bacteria may comprise, for instance, a reporter gene, a selective marker, a gene encoding an enzyme, or a promoter (including an inducible or repressible promoter). The additional gene or regulatory DNA sequence may be integrated into the bacterial chromosome; the additional gene or regulatory DNA sequence may be situated on a plasmid.

The AOBs may differ by at least one nucleotide from naturally occurring bacteria. For instance, the AOBs may differ from naturally occurring bacteria in a gene or protein that is part of a relevant pathway, *e.g*., an ammonia metabolism pathway, a urea metabolism pathway, or a pathway for producing nitric oxide or nitric oxide precursors. More particularly, the AOBs may comprise a mutation that elevates activity of the pathway, *e.g*., by increasing levels or activity of an element of that pathway.

The above-mentioned mutations can be introduced using any suitable technique. Numerous methods are known for introducing mutations into a given position. For instance, one could use site-directed mutagenesis, oligonucleotide-directed mutagenesis, or site-specific mutagenesis. Non-limiting examples of specific mutagenesis protocols are described in, *e.g.,* Mutagenesis, pp. 13.1-13.105 (Sambrook and Russell, eds., Molecular Cloning A Laboratory Manual, Vol. 3, 3.sup.rd ed. 2001). In addition, non-limiting examples of well-characterized mutagenesis protocols available from commercial vendors include, without limitation, Altered Sites.RTM. II in vitro Mutagenesis Systems (Promega Corp., Madison, Wis.); Erase-aBase.RTM. System (Promega, Madison, Wis.); GeneTailor.TM. Site-Directed Mutagenesis System (Invitrogen, Inc., Carlsbad, Calif.); QuikChange.RTM. II Site-Directed Mutagenesis Kits (Stratagene, La Jolla, Calif.); and Transformer.TM. Site-Directed Mutagenesis Kit (BD-Clontech, Mountain View, Calif.).

The ammonia oxidizing microorganisms may be axenic. The preparation (formulation or composition) of ammonia oxidizing microorganisms may comprise, consist essentially of, or consist of axenic ammonia oxidizing microorganisms.

The ammonia oxidizing bacteria of this disclosure may be from a genus selected from the group consisting of *Nitrosomonas, Nitrosococcus, Nitrosospria, Nitrosocystis, Nitrosolobus, Nitrosovibrio,* and combinations thereof.

This disclosure provides, *inter alia, N. eutropha* strain D23, a unique, *e.g.,* optimized strain of ammonia oxidizing bacteria that can increase production of nitric oxide and nitric oxide precursors on a surface of a subject, *e.g*., a human subject. This disclosure also provides methods of administering and using the bacteria and preparations, compositions, formulations, and products, comprising the bacteria.

The ammonia oxidizing bacteria, e.g., *N. eutropha* may be non-naturally occurring. For instance, it may have accumulated desirable mutations during a period of selection. Desirable mutations may be introduced by an experimenter. The *N. eutropha* may be a purified preparation, and may be an optimized *N. eutropha.*

The *N. eutropha* strain may be autotrophic and so incapable of causing infection. A preferred strain utilizes urea as well as ammonia, so that hydrolysis of the urea in sweat would not be necessary prior to absorption and utilization by the bacteria. Also, in order to grow at low pH, the bacteria may either absorb NH₄⁺ ions or urea. The selected strain should also be capable of living on the external skin of a subject, *e.g.*, a human, and be tolerant of conditions there.

Although this disclosure refers to *N. eutropha* strain D23 in detail, the preparations, methods, compositions, treatments, formulas and products may be used with one or more of: one or more other strains of *N. eutropha,* one or more other species of *Nitrosomonas,* and one or more other ammonia oxidizing microorganism, e.g. ammonia oxidizing bacteria or other ammonia oxidizing archaea.

A bacterium with the above-mentioned sequence characteristics may have one or more of (1) an optimized growth rate as measured by doubling time, (2) an optimized growth rate as measured by OD600, (3) an optimized NH₄⁺ oxidation rate, (4) an optimized resistance to NH₄⁺, and (4) an optimized resistance to NO₂⁻. Particular nonlimiting subcombinations of these properties are specified in the following paragraph.

The ammonia oxidizing bacteria, e.g., the *N. eutropha* described herein, or an axenic composition thereof, may have one or more of: (1) an optimized growth rate as measured by doubling time, (2) an optimized growth rate as measured by OD600, (3) an optimized NH₄⁺ oxidation rate, (4) an optimized resistance to, NH₄⁺, and (4) an optimized resistance to, NO₂⁻. For instance, the bacterium may have properties (1) and (2); (2) and (3); (3) and (4); or (4) and (5) from the list at the beginning of this paragraph. As another example, the bacterium may have properties (1), (2), and (3); (1), (2), and (4); (1), (2), and (5); (1), (3), and (4); (1), (3), and (5); (1), (4), and (5); (2), (3), and (4); (2), (3), and (5), or (3), (4), and (5) from the list at the beginning of this paragraph. As a further example, the bacterium may have properties (1), (2), (3), and (4); (1), (2), (3), and (5); (1), (2), (4), and (5); (1), (3), (4), and (5); or (2), (3), (4), and (5) from the list at the beginning of this paragraph. The bacterium may have properties (1), (2), (3), (4), and (5) from the list at the beginning of this paragraph.

The *N. eutropha* strain may comprise a nucleic acid sequence, *e.g.,* a genome, that hybridizes to SEQ ID NO: 1 of International (PCT) Patent Application Publication No. WO2015160911 (International (PCT) Patent Application Serial No. PCT/US2015/025909 filed on April 15, 2015), or to the genome of the D23 strain deposited in the form of 25 vials with the ATCC patent depository on April 8, 2014, designated AOB D23-100, under accession number PTA-121157, or their complements, under low stringency, medium stringency, high stringency, or very high stringency, or other hybridization condition.

The D23 strain is not believed to be a product of nature, but rather has acquired certain mutations and characteristics during an extended period of culture and selection in the laboratory. For instance, D23 has an ability to grow in conditions of greater than about 200 or 250 mM NH₄⁺ for more than 24 hours.

The *N. eutropha* disclosed herein may differ from naturally occurring bacteria in the abundance of siderophores. For instance, the *N. eutropha* may have elevated or reduced levels of siderophores compared to *N. eutropha* C91. Generally, siderophores are secreted iron-chelating compounds that help bacteria scavenge iron from their environment. Some siderophores are peptides, and others are small organic molecules.

The practice of the present disclosure may employ, unless otherwise indicated, conventional methods of immunology, molecular biology, and recombinant DNA techniques within the skill of the art. Such techniques are explained fully in the literature. See, e.g., Sambrook, et al. Molecular Cloning: A Laboratory Manual (Current Edition); and Current Protocols in Molecular Biology (F.M. Ausubel, et al. eds., current edition).

### Select Definitions

An ammonia oxidizing microorganism, e.g., ammonia oxidizing bacteria, refers to a microorganism capable of oxidizing ammonia or ammonium to nitrite at a rate, *e.g.,* a substantial rate, *e.g.,* a pre-determined rate. The rate, *e.g.,* a pre-determined rate, may refer to the conversion of ammonium ions (NH₄⁺) (*e.g.,* at about 200 mM) to nitrite (NO₂⁻), for example, as determined or measured in an *in vitro* assay or when administered to a subject, e.g., a human. The rate may be a conversion at a rate of at least about 1 picomole per minute per mg protein, 0.01, 0.1, 1, 10, 25, 50, 75, 125, or 150 nanomoles NO₂⁻ per minute per mg protein, e.g., about 0.01-1, 0.1-50, 50-100, 100-150, 75-175, 75-125, 100-125, 125-150, or 125-175 nanomoles/minute/mg protein, e.g., about 125 nanomoles NO₂⁻ per minute per mg protein for a continuous culture, for example having an OD of about 0.5. The rate of conversion may be between about 1 picomole per minute per mg protein to about 1 millimole per minute per mg protein. The rate of conversion may be at most about 1 mole NO₂⁻ per minute per mg protein, e.g. at least about, about, or at most about 1 decimole, 1 centimole, 1 millimole, or 1 micromole NO₂⁻ per minute per mg protein.

As used herein, "axenic" refers to a composition comprising an organism that is substantially free of other organisms. For example, an axenic culture of ammonia oxidizing bacteria is a culture that is substantially free of organisms other than ammonia oxidizing bacteria. For example, an axenic culture of *N. eutropha* is a culture that is substantially free of organisms other than *N. eutropha.* "Substantially free" may denote undetectable by a method used to detect other organisms, *e.g*., plating the culture and examining colony morphology, or PCR for a conserved gene such as 16S RNA. An axenic composition may comprise elements that are not organisms, *e.g*., it may comprise nutrients or excipients. Any preparation, composition, or formulation of ammonia oxidizing bacteria discussed
herein may comprise, consist essentially of, or consist of optionally axenic ammonia oxidizing bacteria.

Throughout this disclosure, formulation may refer to a composition or preparation or product.

As used herein, an "autotroph", *e.g*., an autotrophic bacterium, is any organism capable of self-nourishment by using inorganic materials as a source of nutrients and using photosynthesis or chemosynthesis as a source of energy. Autotrophic bacteria may synthesize organic compounds from carbon dioxide and ATP derived from other sources, oxidation of ammonia to nitrite, oxidation of hydrogen sulfide, and oxidation of Fe²⁺ to Fe³⁺. Autotrophic bacteria of the present disclosure are incapable of causing infection.

Administered "in combination," as used herein, means that two (or more) different treatments are delivered to the subject during the course of the subject's affliction with the disorder, *e.g.,* the two or more treatments are delivered after the subject has been diagnosed with the disorder and before the disorder has been cured or eliminated. The delivery of one treatment may still be occurring when the delivery of the second begins, so that there is overlap. This is sometimes referred to herein as "simultaneous" or "concomitant" or "concurrent delivery". The delivery of one treatment may end before the delivery of the other treatment begins. This is sometimes referred to herein as "successive" or "sequential delivery." In either case, the treatment may be more effective because of combined administration. For example, the second treatment is a more effective, *e.g*., an equivalent effect is seen with less of the second treatment, or the second treatment reduces symptoms to a greater extent, than would be seen if the second treatment were administered in the absence of the first treatment, or the analogous situation is seen with the first treatment. Delivery may be such that the reduction in a symptom, or other parameter related to the disorder is greater than what would be observed with one treatment delivered in the absence of the other. The effect of the two treatments can be partially additive, wholly additive, or greater than additive (*i.e.,* synergistic). The delivery can be such that an effect of the first treatment delivered is still detectable when the second is delivered. One or more treatment may be delivered prior to diagnosis of the patient with the disorder.

The term "isolated," as used herein, refers to material that is removed from its original or native environment (*e.g*., the natural environment if it is naturally occurring). For example, a naturally-occurring polynucleotide or polypeptide present in a living animal is not isolated, but the same polynucleotide or polypeptide, separated by human intervention from some or all of the co-existing materials in the natural system, is isolated. Such polynucleotides could be part of a vector and/or such polynucleotides or polypeptides could be part of a composition, and still be isolated in that such vector or composition is not part of the environment in which it is found in nature.

As used herein, the term "optimized growth rate" refers to one or more of: a doubling time of less than about 4, 5, 6, 7, 8, 9, or 10 hours when cultured under batch conditions as described herein in Example 2; a doubling time of less than about 16, 18, 20, 22, 24, or 26 hours, when grown under chemostat conditions as described herein in Example 2; or growing from an OD600 of about 0.15 to at least about 0.3, 0.4, 0.5, 0.6, 0.7, or 0.8 over about 1 or 2 days. Optimized growth rate may be one having a doubling time that it is at least 10, 20, 30, 40, or 50% shorter than that of a naturally occurring *N. eutropha.*

As used herein, "optimized NH₄⁺ oxidation rate" refers to a rate of at least about 50, 75, 125, or 150 micromoles per minute of converting NH₃ or NH₄⁺ into NO₂⁻. For instance, the rate may be at least about 50, 75, 125, or 150 micromoles per minute of converting NH₄⁺ (*e.g.,* at about 200 mM) to NO₂⁻. An optimized NH₄⁺ oxidation rate may be one in which NH₃ or NH₄⁺ is converted into NO₂^{-,} at least 10, 20, 30, 40, or 50% more rapidly than is seen with a naturally occurring *N. eutropha.*

As used herein, "optimized resistance to NH₄⁺" refers to an ability to grow in conditions of greater than 50, 75, 100, 125, 150, 175, 200, 225, 250, 275, or 300 mM NH₃ or NH₄⁺ for at least about 24 or 48 hours. An optimized resistance to NH₄⁺ may refer to the ability to grow at least 10, 20, 30, 40, or 50% more rapidly, or at least 10, 20, 30, 40, or 50% longer, in the presence of a selected concentration of NH₃ or NH₄⁺ than can a naturally occurring *N. eutropha.*

As used herein, "transgenic" means comprising one or more exogenous portions of DNA. The exogenous DNA is derived from another organism, *e.g*., another bacterium, a bacteriophage, an animal, or a plant.

As used herein, treatment of a disease or condition refers to reducing the severity or frequency of at least one symptom of that disease or condition, compared to a similar but untreated patient. Treatment can also refer to halting, slowing, or reversing the progression of a disease or condition, compared to a similar but untreated patient. Treatment may comprise addressing the root cause of the disease and/or one or more symptoms.

As used herein a therapeutically effective amount refers to a dose sufficient to prevent advancement, or to cause regression of a disease or condition, or which is capable of relieving a symptom of a disease or condition, or which is capable of achieving a desired result. A therapeutically effective dose can be measured, for example, as a number of bacteria or number of viable bacteria (*e.g.,* in CFUs) or a mass of bacteria (*e.g.,* in milligrams, grams, or kilograms), or a volume of bacteria (*e.g*., in mm³).

As used herein, the term "viability" refers to the autotrophic bacteria's, *e.g*., ammonia oxidizing bacteria's, ability to oxidize ammonia, ammonium, or urea to nitrite at a pre-determined rate. The rate may refer to the conversion of ammonium ions (NH₄⁺) (*e.g.,* at about 200 mM) to nitrite (NO₂⁻) at a rate of at least about 1 picomole, 0.01, 0.1, 1, 10, 25, 50, 75, 125, or 150 nanomoles NO₂⁻ per minute, *e.g*., about 0.01-1, 0.1-50, 50-100, 100-150, 75-175, 75-125, 100-125, 125-150, or 125-175 nanomoles/minute, *e.g*., about 125 nanomoles NO₂⁻ per minute. The rate of conversion may be at most about 1 mole NO₂⁻ per minute, e.g. at least about, about, or at most about 1 decimole, 1 centimole, 1 millimole, or 1 micromole NO₂⁻ per minute. Viable ammonia oxidizing microorganisms may generally comprise culturable AOMs or AOMs that are otherwise able to generate NO, nitrate, or nitrite.

As used herein, a "subject" may include an animal, a mammal, a human, a non-human animal, a livestock animal, or a companion animal. The term "subject" is intended to include human and non-human animals, for example, vertebrates, large animals, and primates. The subject may be a mammalian subject, and in particular, the subject may be a human subject. Although applications with humans are clearly foreseen, veterinary applications, for example, with non-human animals, are also envisaged herein. The term "non-human animals" of the disclosure includes all vertebrates, for example, non-mammals (such as birds, for example, chickens; amphibians; reptiles) and mammals, such as non-human primates, domesticated, and agriculturally useful animals, for example, sheep, dog, cat, cow, pig, rat, among others.

"Microbiome" refers to a population, e.g, one or more microorganisms that live on a surface of a subject, e.g., in the gut, mouth, skin, and/or elsewhere in a subject. The population may have one or more beneficial functions and/or benefits, relevant to supporting the life of a subject.

"Biome-friendly" refers to something, e.g, a product, e.g., a cosmetic product, e.g., a finished cosmetic product that may allow for minimal disruption of a microbiome of a subject. For example, biome-friendly refers to a product that may be applied to a subject that may allow the microbiome at the point of application to be maintained, minimally disrupted, and/or able to return to the microbiome after a period of time after application of the product. Biome-friendly may refer to ammonia oxidizing microorganism-friendly, e.g. ammonia oxidizing bacteriafriendly in that the product may allow for minimal disruption of the ammonia oxidizing bacteria of a subject. "Biome-friendly" may be referred to as "biome-compatible."

A "natural product" is or may comprise a product that may be at least partially derived from nature. It may be anything or comprise anything produced by a living organism, and may include organisms themselves. Natural products may include or comprise an entire organism, and part of an organism (*e.g.*, a leaf of a plant), an extract from an organism, an organic compound from an organism, a purified organic compound from an organism. Natural products may be or comprise organic substances found and cells, including primary metabolites (amino acids, carbohydrates, and nucleic acids) and secondary metabolites (organic compounds found in a limited range of species, e.g., polyketides, fatty acids, terpenoids, steroids, phenylpropanoids, alkaloids, specialized amino acids and peptides, specialized carbohydrates). Natural products may be or comprise polymeric organic materials such as cellulose, lignin, and proteins.

As used herein, "presence" or "level" may refer to a qualitative or quantitative amount of a component, *e.g*., any one or more of an ammonia oxidizing microorganisms, ammonia, ammonium ions, urea, nitrite, or nitric oxide. The presence or level may include a zero value or a lack of presence of a component.

As used herein, the term "surfactant", includes compounds that may lower the surface tension, or interfacial tension, between two liquids or between a liquid and a solid. Surfactants may act as detergents, wetting agents, emulsifiers, foaming agents, and dispersants. Surfactants may include one or more of the following, alone. or in combination with those listed, or other surfactants or surfactant-like compounds: cocamidopropyl betaine (ColaTeric COAB), polyethylene sorbitol ester (*e.g*., Tween 80), ethoxylated lauryl alcohol (RhodaSurf 6 NAT), sodium laureth sulfate/lauryl glucoside/cocamidopropyl betaine (Plantapon 611 L UP), sodium laureth sulfate (*e.g.,* RhodaPex ESB 70 NAT), alkyl polyglucoside (*e.g.,* Plantaren 2000 N UP), sodium laureth sulfate (Plantaren 200), Dr. Bronner's Castile soap, Dr. Bronner's baby soap, Lauramine oxide (ColaLux Lo), sodium dodecyl sulfate (SDS), polysulfonate alkyl polyglucoside (PolySufanate 160 P), sodium lauryl sulfate (Stepanol-WA Extra K), and combinations thereof. Dr. Bronner's Castile soap and baby soap comprises water, organic coconut oil, potassium hydroxide, organic olive oil, organic fair deal hemp oil, organic jojoba oil, citric acid, and tocopherol. Surfactants may include Sodium Laurylglucosides Hydroxypropylsulfonate (Suga^{®}nate 160NC), lauramidopropyl betaine (Cola^{®}Teric LMB); Cocamidopropyl hydroxysultaine (Cola^{®}Teric CBS); disodium cocoamphodiacetate (Cola^{®}Teric CDCX-LV); sodium laurylglucosides hydroxypropyl phosphate (Suga^{®}Fax D12). Surfactants may include sodium lauroyl methyl isethionate (Iselux^{®} LQ-CLR-SB); sodium methyl cocoyl taurate (Pureact WS Conc.); Aqua (and) Sodium Lauroyl Methyl Isethionate (and) Cocamidopropyl Betaine (and) Sodium Cocoyl Isethionate (and) Sodium Methyl Oleoyl Taurate (Iselux ^{®}SFS-SB). Other surfactants are contemplated by this disclosure.

### Preparations, Compositions, Formulations, and Products Comprising Ammonia Oxidizing Microorganisms

The present disclosure provides, *inter alia,* compositions comprising ammonia oxidizing microorganisms, preparations, e.g., purified and/or optimized preparations, comprising AOM, formulations comprising AOM, and various products comprising AOM, *e.g.,* a natural product, a non-natural product, a fortified natural product, a consumer product, a therapeutic product, or a cosmetic product. The terms preparation, composition, formulation, and product may be used interchangeably herein.

Any preparation, composition, formulation, or product of ammonia oxidizing microorganisms discussed herein may comprise, consist essentially of, or consist of (optionally axenic) ammonia oxidizing microorganisms, e.g., live ammonia oxidizing microorganisms.

The preparation may comprise or be supplemented with a product or byproduct of an ammonia oxidizing microorganism, e.g., nitrite, nitrate, nitric oxide, CoQ8. The preparation may comprise or be supplemented with a composition that promotes growth or metabolism of ammonia oxidizing microorganisms, promotes production of products or byproducts of ammonia oxidizing microorganisms, promotes urease activity, or has a synergistic effect with ammonia oxidizing microorganisms, e.g., ammonia, ammonium salts, urea, and urease. For instance, the preparation may be supplemented with one or more of NO, nitrite, nitrate, CoQ8, ammonia, ammonium salts, urea, and urease. The supplement may be comprised in the same formulation as the ammonia oxidizing microorganisms or in a separate formulation for concurrent or combination administration. The supplement formulation may be prepared for delivery via any delivery mode, for example inhaled forms of NO, nitrite, or nitrate. The preparation may comprise, *inter alia,* at least one of ammonia, ammonium salts, and urea. The preparation may comprise or be supplemented with an anti-inflammatory agent or a composition that provides an anti-inflammatory effect.

The present disclosure provides for preparations comprising ammonia oxidizing microorganisms for cosmetic use.

The present disclosure provides for preparations comprising ammonia oxidizing microorganisms for therapeutic use.

A preparation of ammonia oxidizing microorganisms may comprise a concentration or amount, e.g., an effective amount, of ammonia oxidizing microorganisms sufficient to have a desired cosmetic effect. The preparation may be formulated and/or delivered to impart the desired cosmetic effect locally and/or systemically.

A preparation of ammonia oxidizing microorganisms may comprise a concentration or amount, e.g., an effective amount, of ammonia oxidizing microorganisms sufficient to have a desired therapeutic effect, e.g., to at least partially treat a
condition or disease. The preparation may be formulated and/or delivered to impart the desired therapeutic effect locally and/or systemically.

A preparation of ammonia oxidizing microorganisms may comprise a concentration or amount, e.g., an effective amount, of ammonia oxidizing microorganisms sufficient to alter, *e.g.,* reduce or increase, an amount, concentration or proportion of a bacterium, or genus of bacteria in a subject. The bacteria may be non-pathogenic or pathogenic, or potentially pathogenic.

A preparation of ammonia oxidizing microorganisms may comprise a concentration or amount, e.g., an effective amount, of ammonia oxidizing microorganisms sufficient to modulate a microbiome associated with a subject.

A preparation of ammonia oxidizing microorganisms may comprise a concentration or amount, e.g., an effective amount, of ammonia oxidizing microorganisms sufficient to deliver NO to a subject. A preparation of ammonia oxidizing microorganisms may comprise a concentration or amount, e.g., an effective amount, of ammonia oxidizing microorganisms such that when administered, the preparation modulates, changes, or alters a level of nitrite or NO at a target tissue or in circulation. For instance, a preparation of ammonia oxidizing microorganisms may comprise a concentration or amount, e.g., an effective amount, of ammonia oxidizing microorganisms such that when administered, the preparation results in an increased level of nitrite or NO at a target tissue or in circulation.

The present disclosure provides, *inter alia,* non-limiting compositions comprising ammonia oxidizing microorganisms, e.g., *N. eutropha,* e.g., a purified preparation of an optimized *N. eutropha.* The *N. eutropha* in the compositions may have at least one property selected from an optimized growth rate, an optimized NH₄⁺ oxidation rate, and an optimized resistance to NH₄⁺.

The present disclosure provides compositions with a defined number of species. A composition may include only one type of species, e.g., one type of ammonia oxidizing microorganism. This disclosure also provides a composition having, e.g., *N. eutropha* and one other type of organism, and no other types of organism. In other examples, the composition has, e.g., *N. eutropha* and 2, 3, 4, 5, 6, 7, 8, 9, or 10 other types of organism, and no other types of organism. The other type of organism in this composition may be, for instance, a bacterium, such as an ammonia-oxidizing bacterium. Suitable ammonia-oxidizing microorganisms for this purpose include those in the genera *Nitrosomonas, Nitrosococcus, Nitrosospira, Nitrosocystis, Nitrosolobus,* or *Nitrosovibrio.* Likewise, the composition may also include AOA.

The composition comprising, e.g., *N. eutropha* may provide conditions that support*N*. *eutropha* viability. For instance, the composition may promote *N. eutropha* growth and metabolism or may promote a dormant state (*e.g.,* freezing) from which viable *N. eutropha* can be recovered. When the composition promotes growth or metabolism, it may contain water and/or nutrients that *N. eutropha* consumes, *e.g.,* as ammonium, ammonia, urea, oxygen, carbon dioxide, or trace minerals. The composition comprising ammonia oxidizing microorganisms may provide conditions that support ammonia oxidizing microorganisms viability. For instance, the composition may promote ammonia oxidizing microorganisms growth and metabolism or may promote a dormant state (*e.g.,* freezing) or storage state as described herein, from which viable ammonia oxidizing microorganisms can be recovered. When the composition promotes growth or metabolism, it may contain water and/or nutrients that ammonia oxidizing microorganisms consumes, *e.g*., as ammonium ions, ammonia, urea, oxygen, carbon dioxide, or trace minerals.

One or more other organisms, for example, organisms besides ammonia oxidizing microorganisms, may be included in the preparation of ammonia oxidizing microorganisms. For example, a community of organisms or an organism of the genus selected from the group consisting of *Lactobacillus, Streptococcus, Bifidobacter,* and combinations thereof, may be provided in the preparation of ammonia oxidizing microorganisms. The preparation may be substantially free of other organisms.

Preparations of ammonia oxidizing microorganisms may comprise between about between about 10³ to about 10¹⁴ CFU/ml. The preparation of ammonia oxidizing microorganisms may comprise at least about or greater than about 10³, 10⁴, 10⁵, 10⁶, 10⁷, 10⁸, 10⁹, 10¹⁰, 10¹¹, 2 x 10¹¹, 5 x 10¹¹, 10¹², 2 x 10¹² , 5 x 10¹², 10¹³, 2 x 10¹³, 5 x 10¹³, or 10¹⁴; or about 10³-10⁴, 10⁴-10⁵, 10⁶-10⁷, 10⁷-10⁸, 10⁸-10⁹, 10⁹-10¹⁰, 10¹⁰-10¹¹, 10¹¹-10¹², 10¹²-10¹³, or 10¹³-10¹⁴ CFU/ml.

A preparation of ammonia oxidizing microorganisms may comprise between about 1 x 10⁹ to about 10 x 10⁹ CFU/ml. An administered dose of the preparation may comprise about 3 x 10¹⁰ CFU, *e.g.,* 3 x 10¹⁰ CFU per day. An administered dose of the preparation may comprise about 1 x 10⁹ to about 10 x 10⁹ CFU per day, e.g., about 1 x 10⁹ to about 10 x 10⁹ CFU per day. An administered dose of the preparation may comprise about 10³, 10⁴, 10⁵, 10⁶, 10⁷, 10⁸, 10⁹, 10¹⁰, 10¹¹, 2 x 10¹¹, 5 x 10¹¹, 10¹², 2x 10¹² , 5 x 10¹², 10¹³, 2 x 10¹³, 5 x 10¹³, or 10¹⁴; or about 10³-10⁴, 10⁴-10⁵, 10⁶-10⁷, 10⁷-10⁸, 10⁸-10⁹, 10⁹-10¹⁰, 10¹⁰-10¹¹, 10¹¹-10¹², 10¹²-10¹³, or 10¹³-10¹⁴ CFU per administration or per day.

An administered dose of the preparation may comprise at least about 7 x 10¹⁰ CFU, *e.g.,* 21 x 10¹⁰ CFU per week. An administered dose of the preparation may comprise about 1 x 10⁹ to about 10 x 10⁹ CFU per week, e.g., about 1 x 10⁹ to about 10 x 10⁹ CFU per week. An administered dose of the preparation may comprise about or greater than about 10³, 10⁴, 10⁵, 10⁶, 10⁷, 10⁸, 10⁹ , 10¹⁰, 10¹¹, 2 x 10¹¹, 5 x 10¹¹, 10¹², 2 x 10¹² , 5 x 10¹², 10¹³, 2 x 10¹³, 5 x 10¹³, or 10¹⁴; or about 10³-10⁴, 10⁴-10⁵, 10⁶-10⁷, 10⁷-10⁸, 10⁸-10⁹, 10⁹-10¹⁰, 10¹⁰-10¹¹, 10¹¹-10¹², 10¹²-10¹³, or 10¹³-10¹⁴ CFU per week.

An administered dose of the preparation may comprise at least about 30 x 10¹⁰ CFU, *e.g.,* 90 x 10¹⁰ CFU per month. An administered dose of the preparation may comprise about 1 x 10⁹ to about 10 x 10⁹ CFU per month, e.g., about 1 x 10⁹ to about 10 x 10⁹ CFU per month. An administered dose of the preparation may comprise about or greater than about 10³, 10⁴, 10⁵, 10⁶, 10⁷, 10⁸, 10⁹, 10¹⁰, 10¹¹, 2 x 10¹¹, 5 x 10¹¹, 10¹² , 2x 10¹² , 5 x 10¹², 10¹³, 2x 10¹³, 5 x 10¹³, or 10¹⁴; or about 10³-10⁴, 10⁴-10⁵, 10⁶-10⁷, 10⁷-10⁸, 10⁸-10⁹, 10⁹-10¹⁰, 10¹⁰-10¹¹, 10¹¹-10¹², 10¹²-10¹³, or 10¹³-10¹⁴ CFU per month.

The preparation of ammonia oxidizing microorganisms may comprise between about 0.1 milligrams (mg) and about 1000 mg of ammonia oxidizing microorganisms. The preparation may comprise between about 50 mg and about 1000 mg of ammonia oxidizing microorganisms. The preparation may comprise between about 0.1-0.5 mg, 0.2-0.7 mg, 0.5-1.0 mg, 0.5-2 mg, 0.5-5 mg, 2.5-5 mg, 2.5-7.0 mg, 5.0-10 mg,
7.5-15 mg, 10-15 mg, 15-20 mg, 15-25 mg, 20-30 mg, 25-50 mg, 25-75 mg, 50-75 mg, 50-100 mg, 75-100 mg, 100-200 mg, 200-300 mg, 300-400 mg, 400-500 mg, 500-600 mg, 600-700 mg, 700-800 mg, 800-900 mg, 900-1000 mg, 100-250 mg, 250-500 mg, 100-500 mg, 500-750 mg, 750-1000 mg, or 500-1000 mg.

Advantageously, a formulation may have a pH level that promotes AOM, *e.g., N. eutropha* viability, *e.g.,* metabolic activity. Urea would hydrolyze to ammonia and would raise the pH to 7 to 8. AOB are very active at this pH range and would lower the pH to about 6 where the NH₃ converts to ammonium and is unavailable. Lower pH levels, e.g. about pH 4, are also acceptable.

The ammonia oxidizing microorganisms, *e.g., N. eutropha* may be combined with one or more pharmaceutically or cosmetically acceptable excipients. "Pharmaceutically acceptable excipient" may refer to a pharmaceutically-acceptable material, composition, or vehicle, such as a liquid or solid filler, diluent, solvent, or encapsulating material. Each excipient may be "pharmaceutically acceptable" in the sense of being compatible with the other ingredients of a pharmaceutical formulation, and suitable for use in contact with the tissue or organ of humans and animals without excessive toxicity, irritation, allergic response, immunogenicity, or other problems or complications, commensurate with a reasonable benefit/risk ratio. See, Remington: The Science and Practice of Pharmacy, 21st ed.; Lippincott Williams & Wilkins: Philadelphia, Pa., 2005; Handbook of Pharmaceutical Excipients, 6th ed.; Rowe et al., Eds.; The Pharmaceutical Press and the American Pharmaceutical Association: 2009; Handbook of Pharmaceutical Additives, 3rd ed.; Ash and Ash Eds.; Gower Publishing Company: 2007; Pharmaceutical Preformulation and Formulation, 2nd ed.; Gibson Ed.; CRC Press LLC: Boca Raton, Fla., 2009.

A cosmetically acceptable excipient may refer to a cosmetically acceptable material, composition, or vehicle, such as a liquid or solid filler, diluent, solvent, or encapsulating material. Each excipient may be cosmetically acceptable in the sense of being compatible with the other ingredients of a cosmetic formulation, and suitable for use in contact with the tissue or organ of humans and animals without excessive toxicity, irritation, allergic response, immunogenicity, or other problems or complications, commensurate with a reasonable benefit/risk ratio.

While it is possible for the active ingredient, e.g., ammonia oxidizing microorganisms, *e.g., N. eutropha,* to be administered alone, it may be present in a pharmaceutical formulation or composition. Accordingly, this disclosure provides a pharmaceutical formulation comprising ammonia oxidizing microorganisms, for example, *N. eutropha* and a pharmaceutically acceptable excipient. Pharmaceutical compositions may take the form of a pharmaceutical formulation as described below.

A preparation of ammonia oxidizing microorganisms may be formulated in order to facilitate a desired delivery mechanism or mode of administration thereof. The formulations, e.g., pharmaceutical or cosmetic formulations, described herein include those suitable for, e.g., oral, enteral (including buccal, sublingual, sublabial, and rectal), parenteral (including subcutaneous, intradermal, intramuscular, intravenous, and intraarticular), inhalation (including fine particle dusts or mists which may be generated by means of various types of metered doses, pressurized aerosols, nebulizers or insufflators, and including intranasally or via the lungs), intranasal, eye, ear, rectal, injection, urogenital, and topical (including dermal, transdermal, transmucosal, buccal, sublingual, and intraocular) administration, although the most suitable route may depend upon, for example, a condition or disorder of a recipient.

A preparation comprising ammonia oxidizing microorganisms may be administered to a subject, e.g., for cosmetic or therapeutic purposes, as a solution, suspension, powder, liquid, drop, spray, aerosol, mist, emulsion, foam, cream, ointment, gel, hydrogel, resin, tablet, capsule, film, suppository, enema, douche, pessary, insert, patch, e.g., transdermal patch, or implantable device, e.g., stent, catheter, vaginal ring, or intrauterine device.

Devices configured to deliver a preparation comprising live ammonia oxidizing microorganisms via a desired mode of administration or otherwise via targeted delivery are also disclosed.

The preparation may be formulated for targeted delivery to a subject, e.g., to a target tissue, region, system, or organ of a subject. For example, the preparation may be formulated for delivery to the eye, ear, nose, urogenital system, respiratory system, or gastrointestinal system of the subject. Targeted
delivery may be based on a condition or disorder of a subject. For instance, formulation for targeted delivery may be based on a desired local or systemic effect to be achieved, e.g., a local or systemic therapeutic or cosmetic effect. A target tissue, region, system, or organ of a subject may be selected for its association with a desired local or systemic effect.

The formulations may conveniently be presented in unit dosage form and may be prepared by any of the methods known in the art of pharmacy. Typically, methods include the step of bringing the active ingredient (*e.g.,* ammonia oxidizing microorganisms, *e.g., N. eutropha*) into association with a pharmaceutical carrier which constitutes one or more accessory ingredients. In general the formulations are prepared by uniformly and intimately bringing into association the active ingredient with liquid carriers or finely divided solid carriers or both and then, if necessary, shaping the product into the desired formulation.

Formulations may be presented as discrete units such as capsules, cachets or tablets, each containing a predetermined amount of, *e.g., N. eutropha;* as a powder or granules; as a solution or a suspension in an aqueous liquid or a non-aqueous liquid; or as an oil-in-water liquid emulsion or a water-in-oil liquid emulsion. Formulations, e.g., solutions, aerosols, sprays, and mists, may be presented in multi-dosage form, e.g., packaged units including a predetermined number of dosages, or single dosage form, e.g., packaged units including a single dose. The active ingredient may also be presented as a bolus, electuary or paste. Various pharmaceutically acceptable carriers and their formulation are described in standard formulation treatises, *e.g*., Remington's Pharmaceutical Sciences by E. W. Martin. See also Wang, Y. J. and Hanson, M. A., Journal of Parenteral Science and Technology, Technical Report No. 10, Supp. 42:2 S, 1988.

The ammonia oxidizing microorganisms, *e.g., N. eutropha* compositions can, for example, be administered in a form suitable for immediate release or extended release. Suitable examples of sustained-release systems include suitable polymeric materials, for example semipermeable polymer matrices in the form of shaped articles, *e.g*., films, or microcapsules; suitable hydrophobic materials, for example as an emulsion in an acceptable oil; or ion exchange resins. Sustained-release systems may be administered orally; rectally; parenterally; intracisternally; intravaginally; intraperitoneally; topically, for example as a powder, ointment, gel, drop or transdermal patch; bucally; or as a spray.

Preparations for administration can be suitably formulated to give controlled release of ammonia oxidizing microorganisms, *e.g., N. eutropha.* For example, the pharmaceutical compositions may be in the form of particles comprising one or more of biodegradable polymers, polysaccharide jellifying and/or bioadhesive polymers, or amphiphilic polymers. These compositions exhibit certain biocompatibility features which allow a controlled release of an active substance. See U.S. Pat. No. 5,700,486.

Exemplary compositions include suspensions which can contain, for example, microcrystalline cellulose for imparting bulk, alginic acid or sodium alginate as a suspending agent, methylcellulose as a viscosity enhancer, dicalcium phosphate, starch, magnesium stearate and/or lactose and/or other excipients, binders, extenders, disintegrants, diluents and lubricants, mannitol, lactose, sucrose and/or cyclodextrins. Also included in such formulations may be high molecular weight excipients such as celluloses (avicel) or polyethylene glycols (PEG). Such formulations can also include an excipient to aid mucosal adhesion such as hydroxy propyl cellulose (HPC), hydroxy propyl methyl cellulose (HPMC), sodium carboxy methyl cellulose (SCMC), maleic anhydride copolymer (*e.g*., Gantrez), and agents to control release such as polyacrylic copolymer (*e.g*. Carbopol 934). Lubricants, glidants, flavors, coloring agents and stabilizers may also be added for ease of fabrication and use. The surfactant may be a zwitterionic surfactant, a non-ionic surfactant, or an anionic surfactant.

Excipients, such as surfactants that may be used with preparations of the present disclosure may include one or more of cocamidopropyl betaine (ColaTeric COAB), polyethylene sorbitol ester (*e.g*., Tween 80), ethoxylated lauryl alcohol (RhodaSurf 6 NAT), sodium laureth sulfate/lauryl glucoside/cocamidopropyl betaine (Plantapon 611 L UP), sodium laureth sulfate (*e.g.,* RhodaPex ESB 70 NAT), alkyl polyglucoside (*e.g.,* Plantaren 2000 N UP), sodium laureth sulfate (Plantaren 200), Dr. Bronner's Castile soap, Dr. Bronner's Castile baby soap, Lauramine oxide (ColaLux Lo), sodium dodecyl sulfate (SDS), polysulfonate alkyl polyglucoside (PolySufanate 160 P), sodium lauryl sulfate (Stepanol-WA Extra K), and combinations thereof. Dr. Bronner's Castile soap and Dr. Bronner's baby soap comprises water, organic coconut oil, potassium hydroxide, organic olive oil, organic fair deal hemp oil, organic jojoba oil, citric acid, and tocopherol.

Surfactants may be used with ammonia oxidizing microorganisms in amounts that allow nitrite production to occur. The preparation may have less than about 0.0001 % to about 10% of surfactant. The preparation may have between about 0.1 % and about 10 % surfactant. The concentration of surfactant used may be between about 0.0001% and about 10%. The preparation may be substantially free of surfactant.

The formulation, *e.g*., preparation, may include other components that may enhance effectiveness of ammonia oxidizing microorganisms, delivery thereof, or enhance a treatment or indication.

A chelator may be included in the preparation. A chelator may be a compound that may bind with another compound, *e.g*., a metal. The chelator may provide assistance in removing an unwanted compound from an environment, or may act in a protective manner to reduce or eliminate contact of a particular compound with an environment, *e.g*., ammonia oxidizing microorganisms, *e.g.* a preparation of ammonia oxidizing microorganisms, *e.g.,* an excipient. The preparation may be substantially free of chelator.

Formulations may also contain anti-oxidants, buffers, bacteriostats that prevent the growth of undesired microorganisms, solutes, and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of a sterile liquid carrier, for example saline or water-for-injection, immediately prior to use. Extemporaneous solutions and suspensions may be prepared from powders, granules and tablets of the kind previously described. Exemplary compositions include solutions or suspensions which can contain, for example, suitable non-toxic, pharmaceutically acceptable diluents or solvents, such as mannitol, 1,3-butanediol, water, Ringer's solution, an isotonic sodium chloride solution, or other suitable dispersing or wetting and suspending agents, including synthetic mono- or diglycerides, and fatty acids, including oleic acid, or Cremaphor. An aqueous carrier may be, for example, an isotonic buffer solution at a pH of from about 3.0 to about 8.0, a pH of from about 3.5 to about 7.4, for example from 3.5 to 6.0, for example from 3.5 to about 5.0. Useful buffers include sodium citrate-citric acid and sodium phosphate-phosphoric acid, and sodium acetate/acetic acid buffers. The composition in some instances does not include oxidizing agents.

Excipients that can be included are, for instance, proteins, such as human serum albumin or plasma preparations. If desired, the pharmaceutical composition may also contain minor amounts of non-toxic auxiliary substances, such as wetting or emulsifying agents, preservatives, and pH buffering agents and the like, for example sodium acetate or sorbitan monolaurate. Excipients, *e.g.,* a pharmaceutically acceptable excipient or a cosmetically acceptable excipient, may comprise an anti-adherent, binder, coat, disintegrant, filler, flavor, color, lubricant, glidant, sorbent, preservative, or sweetener. The preparation may be substantially free of excipients.

The preparation may be substantially free of one or more of the compounds or substances listed in the disclosure.

Exemplary compositions for spray, aerosol, or mist administration include solutions in saline, which can contain, for example, benzyl alcohol or other suitable preservatives, absorption promoters to enhance bioavailability, and/or other solubilizing or dispersing agents. Conveniently in compositions for aerosol administration the ammonia oxidizing microorganisms, e.g., *N. eutropha* is delivered in the form of an aerosol spray presentation from a pressurized pack or a nebulizer, with the use of a suitable propellant, *e.g.,* dichlorodifluoro-methane, trichlorofluoromethane, dichlorotetrafluoroethane, carbon dioxide or other suitable gas. In the case of a pressurized aerosol the dosage unit can be determined by providing a valve to deliver a metered amount. Capsules and cartridges of *e.g.,* gelatin can be formulated to contain a powder mix of the *N. eutropha* and a suitable powder base, for example lactose or starch. *N. eutropha* may be administered as an aerosol from a metered dose valve, through an aerosol adapter also known as an actuator. Optionally, a stabilizer is also included, and/or porous particles for deep lung delivery are included (*e.g.,* see U.S. Pat. No. 6,447,743).

Formulations may be presented with carriers such as cocoa butter, synthetic glyceride esters or polyethylene glycol. Such carriers are typically solid at ordinary temperatures, but liquefy and/or dissolve at body temperature to release the ammonia oxidizing bacteria, *e.g., N. eutropha.*

Exemplary compositions for topical administration include a topical carrier such as Plastibase (mineral oil gelled with polyethylene). The composition and/or excipient may be in the form of one or more of a liquid, a solid, or a gel. For example, liquid suspensions may include, but are not limited to, water, saline, phosphate-buffered saline, or an ammonia oxidizing storage buffer. Gel formulations may include, but are not limited to agar, silica, polyacrylic acid (for example Carbopol^{®}), carboxymethyl cellulose, starch, guar gum, alginate or chitosan. The formulation may be supplemented with an ammonia source including, but not limited to ammonium chloride or ammonium sulfate.

An ammonia oxidizing microorganism, *e.g., N. eutropha* composition may be formulated to improve NO penetration, e.g., into the skin or other target tissue. A gel-forming material such as KY jelly or various hair gels would present a diffusion barrier to NO loss to ambient air, and so improve the skin's absorption of NO. The NO level in the skin will generally not greatly exceed 20 nM/L because that level activates GC and would cause local vasodilatation and oxidative destruction of excess NO.

It should be understood that in addition to the ingredients particularly mentioned above, the formulations as described herein may include other agents conventional in the art having regard to the type of formulation in question.

The formulation, *e.g.,* preparation, *e.g.,* composition may be provided in a container, delivery system, or delivery device, having a weight, including or not including the contents of the container, that may be less than about 50, 100, 200, 300, 400, 500, 600, 700, 800, 900, 1000, 1500, or 2000 grams.

Suitable unit dosage formulations are those containing an effective dose, as hereinbefore recited, or an appropriate fraction thereof, of ammonia oxidizing microorganisms, *e.g., N. eutropha.*

A therapeutically effective amount of ammonia oxidizing microorganisms, e.g., *N. eutropha* may be administered as a single pulse dose, as a bolus dose, or as pulse doses administered over time. Thus, in pulse doses, a bolus administration of ammonia oxidizing microorganisms, e.g., *N. eutropha* is provided, followed by a time period wherein ammonia oxidizing microorganisms, e.g., *N. eutropha* is administered to the subject, followed by a second bolus administration. In specific, non-limiting examples, pulse doses are administered during the course of a day, during the course of a week, or during the course of a month.

A preparation of ammonia oxidizing microorganisms, *e.g.,* a formulation, *e.g., a* composition, may be applied for a pre-determined number of days. This may be based, for example, at least in part, on the severity of the condition or disease, the response to the treatment, the dosage applied and the frequency of the dose. For example, the preparation may be applied for about 1-3, 3-5, 5-7, 7-9, 5-10, 10-14, 12-18, 12-21, 21-28, 28-35, 35-42, 42-49, 49-56, 46-63, 63-70, 70-77, 77-84, 84-91 days., for about 1 month, for about 2 months, for about 3 months. The ammonia oxidizing bacteria may be administered for an indefinite period of time, e.g., greater than one year, greater than 5 years, greater than 10 years, greater than 15 years, greater than 30 years, greater than 50 years, greater than 75 years. The preparation may be applied for about 16 days.

A preparation of ammonia oxidizing microorganisms, *e.g.,* a formulation, *e.g., a* composition, may be applied a pre-determined number of times per day. This may be based, for example, at least in part, on the severity of the condition or disease, the response to the treatment, the dosage applied and the frequency of the dose. For example, the preparation may be applied 1, 2, 3, 4, 5, 6, 7, 8, 9, 10, 11, 12, 13, 14, 15, 16, 17, 18, 19, 20, 21, 22, 23, 24 times per day.

The preparation may be applied one time per day. The preparation may be applied two times per day. The preparation may be applied a first pre-determined amount for a certain number of days, and a second pre-determined amount for a certain subsequent number of days. The preparation may be applied for about 16 days.

The preparation may generally be compatible with a physiological environment associated with the subject. Compositions may be formulated to have a substantially neutral pH or a physiological pH, for instance a pH that normally prevails in the target site for intended delivery, administration, or desired effect. Compositions may be formulated to have a pH between about 5.5 and about 8.5. Compositions may be formulated to comprise compatible conditions, e.g., pH, tonicity, with the target site of physiological environment associated with the subject.

The preparation may be formulated for transmucosal delivery and/or circulation, e.g. locally or systemically. The preparation may be formulated such that ammonia oxidizing microorganisms, products thereof, or byproducts thereof (e.g., nitrate, nitrite, NO, or CoQ8) penetrate a deposit or target tissue at least 10%, 20%, 30%, 40%, 50%, 60%, 70%, 80%, 90%, or 100%. The preparation may be formulated such that 10%, 20%, 30%, 40%, 50%, 60%, 70%, 80%, 90%, or 100% of ammonia oxidizing microorganisms, products thereof, or byproducts thereof, penetrate a deposit or target tissue or enter circulation.

The preparation may be in the form of a solution, suspension, emulsion, cream, ointment, gel, hydrogel, or liquid, e.g. drop, spray, aerosol, or mist, tablet, capsule, or device for administration to a subject.

A preparation, composition, formulation, or product comprising ammonia oxidizing microorganisms may undergo quality control and/or testing while it is being made and/or upon its completion. International (PCT) Patent Application Publication No. WO2015/179669 (International (PCT) Patent Application Serial No. PCT/US2015/032017 as filed on May 21, 2015) describes various methods of preparing materials with ammonia oxidizing microorganisms and of testing such materials. For example, one or more parameters such as OD level, pH level, waste level, nutrient level, contaminant level, oxidation rate, nitrite level, protein concentration may be compared against a predetermined value to assess or evaluate a preparation comprising ammonia oxidizing microorganisms.

The present disclosure provides, *inter alia,* a kit comprising preparations of ammonia oxidizing microorganisms, as disclosed herein. Formulations may comprise discrete units, e.g., solid, liquid, or gas formulations of ammonia oxidizing microorganisms. Formulations, e.g., solutions, aerosols, sprays, and mists, may be presented in multi-dosage form (multiple use), e.g., packaged units including a predetermined number of dosages, or single dosage form (single use), e.g., packaged units including a single dose. Preparations of ammonia oxidizing microorganisms may be packaged in devices or containers configured to hold a volume of at least about less than 1 ml, 1 ml, 5 ml, 10 ml, 20 ml, 25 ml, 40 ml, 50 ml, 60 ml, 70 ml, 80 ml, 90 ml, 100 ml, or more than about 100 ml.

Kits may further comprise one or more device for administration of the preparation, for example, syringe, needle, catheter, enema, bulb, pipette (eye or ear dropper), and other devices for drug administration as known in the art. Kits may comprise instructions for use, for example instructions for administration of ammonia oxidizing microorganisms as disclosed herein or instructions for combination therapy including administration of ammonia oxidizing microorganisms. Kits may comprise a second or subsequent composition for administration in conjunction with an ammonia oxidizing preparation, as disclosed herein. For instance, kits may comprise a supplement or composition comprising a product or byproduct of ammonia oxidizing microorganisms, a composition that promotes growth or metabolism of ammonia oxidizing microorganisms, a composition that promotes production of products or byproducts of ammonia oxidizing microorganisms, a composition that promotes urease activity, or a composition that has a synergistic effect with ammonia oxidizing microorganisms, or a composition or pharmaceutical agent that treats, e.g., is approved to treat or commonly used to treat, a relevant disease, disorder, or a symptom of a relevant disease or disorder, for example an anti-inflammatory composition. Kits may comprise "biome-friendly" or "biome-compatible" products as disclosed herein, for example one or more microbiome-compatible cosmetic products. Any of the products contained in the kit may be specifically formulated to treat a target indication and/or formulated for a desired mode of delivery, as described herein.

### Natural Products; Consumer Products

A preparation comprising ammonia oxidizing microorganisms as discussed herein may be a natural product or a consumer product. A preparation of ammonia oxidizing microorganism may instead be used in conjunction with a natural product or consumer product.

Ammonia oxidizing microorganisms, *e.g., N. eutropha* may be associated with a variety of natural products, and examples of such products are set out below. These natural products may be comprised of formulations, compositions, or preparations disclosed throughout this disclosure.

Natural products may be or comprise products for commercial purposes, and may refer to cosmetics, dietary supplements, and foods, e.g., food, food supplements, medical food, food additive, nutraceutical, or drink, produced from natural sources. Natural products may have pharmacological or biological activity that may be of therapeutic benefit, *e.g.,* in treating disease or conditions. Natural products may be included in traditional medicines, treatments for cosmetological purposes, and spa treatments. A natural product referred to herein may comprise any one or more of the components described as a natural product to be incorporated into a preparation or formulation comprising one or more other components, *e.g.,* excipients. The preparation or formulation referred to as a natural product may comprise a natural product defined herein and one or more additional components or ingredients. Any of the compositions, preparations, or formulations discussed throughout this disclosure may be or comprise one or more natural products.

The natural product or the fortified natural product may comprise at least one of mud, water, food-derived products, plant-derived products, extracts, and oils. The natural product or the fortified natural product may be used in a spa treatment. The natural product or the fortified natural product may be incorporated into at least one of a powder, cream, lotion, wrap, scrub, eye mask, facial mask, body mask, aerosol, e.g., mist, spray, salve, wipe, stick, bandage, or soak.

The natural product or fortified natural product may be provided as, or may be disposed in at least one of a baby product, *e.g.,* a baby shampoo, a baby lotion, a baby oil, a baby powder, a baby cream; a bath preparation, *e.g.,* a bath oil, a tablet, a salt, a bubble bath, a bath capsule; an eye makeup preparation, *e.g.,* an eyebrow pencil, an eyeliner, an eye shadow, an eye lotion, an eye makeup remover, a mascara; a fragrance preparation, *e.g.,* a colognes, a toilet water, a perfume, a powder (dusting and talcum), a sachet; hair preparations, *e.g.,* hair conditioners, hair sprays, hair straighteners, permanent waves, rinses, shampoos, tonics, dressings, hair grooming aids, wave sets; hair coloring preparations, *e.g.,* hair dyes and colors, hair tints, coloring hair rinses, coloring hair shampoos, hair lighteners with color, hair bleaches; makeup preparations, *e.g.,* face powders, foundations, leg and body paints, lipstick, makeup bases, rouges, makeup fixatives; manicuring preparations, *e.g.,* basecoats and undercoats, cuticle softeners, nail creams and lotions, nail extenders, nail polish and enamel, nail polish and enamel removers; oral hygiene products, *e.g.,* dentrifices, mouthwashes and breath fresheners; bath soaps and detergents, deodorants, douches, feminine hygiene deodorants; shaving preparations, *e.g.,* aftershave lotions, beard softeners, talcum, preshave lotions, shaving cream, shaving soap; skin care preparations, *e.g.,* cleansing, depilatories, face and neck, body and hand, foot powders and sprays, moisturizing, night preparations, paste masks, skin fresheners; and suntan preparations, *e.g.,* gels, creams, and liquids, and indoor tanning preparations.

Ammonia oxidizing microorganisms, *e.g., N. eutropha* may be associated with a variety of consumer products, and examples of such products are set out below and be comprised of formulations, compositions, or preparations disclosed throughout this disclosure. The ammonia oxidizing bacteria, *e.g., N. eutropha* associated with a product may be admixed with the product, for example, spread evenly throughout the product, and ammonia oxidizing bacteria, *e.g.,* the *N. eutropha* associated with a product may be layered on the product.

The preparation may be disposed in, or provided as, a powder, cosmetic, cream, stick, aerosol, e.g., mist, salve, wipe, or bandage.

Ammonia oxidizing bacteria, *e.g., N. eutropha* may be associated with a powder. Powders are typically small particulate solids that are not attached to each other and that can flow freely when tilted. Exemplary powders for consumer use include talcum powder and some cosmetics (*e.g.,* powder foundation).

The ammonia oxidizing bacteria may be associated with a cosmetic. The cosmetic may be a substance for topical application intended to alter a person's appearance, *e.g.,* a liquid foundation, a powder foundation, blush, or lipstick, and may be referred to as a preparation. The cosmetic may be any substance recited in the Food and Drug Administration regulations, *e.g.,* under 21 C.F.R. § 720.4.

Ammonia oxidizing bacteria, *e.g., N. eutropha* may be associated with a cosmetic. The cosmetic may be a substance for topical application intended to alter a person's appearance, *e.g.,* a liquid foundation, a powder foundation, blush, or lipstick. Other components may be added to these cosmetic preparations as selected by one skilled in the art of cosmetic formulation such as, for example, water, mineral oil, coloring agent, perfume, aloe, glycerin, sodium chloride, sodium bicarbonate, pH buffers, UV blocking agents, silicone oil, natural oils, vitamin E, herbal concentrates, lactic acid, citric acid, talc, clay, calcium carbonate, magnesium carbonate, zinc oxide, starch, urea, and erythorbic acid, or any other excipient known by one of skill in the art, including those disclosed herein.

The preparation, e.g., the cosmetic, may be at least one of a baby product, *e.g.,* a baby shampoo, a baby lotion, a baby oil, a baby powder, a baby cream; a bath preparation, *e.g.,* a bath oil, a tablet, a salt, a bubble bath, a bath capsule; an eye makeup preparation, *e.g.,* an eyebrow pencil, an eyeliner, an eye shadow, an eye lotion, an eye makeup remover, a mascara; a fragrance preparation, *e.g.,* a colognes, a toilet water, a perfume, a powder (dusting and talcum), a sachet; hair preparations, *e.g.,* hair conditioners, hair sprays, hair straighteners, permanent waves, rinses, shampoos, tonics, dressings, hair grooming aids, wave sets; hair coloring preparations, *e.g.,* hair dyes and colors, hair tints, coloring hair rinses, coloring hair shampoos, hair lighteners with color, hair bleaches; makeup preparations, *e.g.,* face powders, foundations, leg and body paints, lipstick, makeup bases, rouges, makeup fixatives; manicuring preparations, *e.g.,* basecoats and undercoats, cuticle softeners, nail creams and lotions, nail extenders, nail polish and enamel, nail polish and enamel removers; oral hygiene products, *e.g.,* dentrifices, mouthwashes and breath fresheners; bath soaps and detergents, deodorants, douches, feminine hygiene deodorants; shaving preparations, *e.g.,* aftershave lotions, beard softeners, talcum, preshave lotions, shaving cream, shaving soap; skin care preparations, *e.g.,* cleansing, depilatories, face and neck, body and hand, foot powders and sprays, moisturizing, night preparations, paste masks, skin fresheners; and suntan preparations, *e.g.,* gels, creams, and liquids, and indoor tanning preparations.

The formulations, compositions, or preparations described herein, may comprise, be provided as, or disposed in at least one of a baby product, *e.g.,* a baby shampoo, a baby lotion, a baby oil, a baby powder, a baby cream; a bath preparation, *e.g.,* a bath oil, a tablet, a salt, a bubble bath, a bath capsule; a powder (dusting and talcum), a sachet; hair preparations, *e.g.,* hair conditioners, rinses, shampoos, tonics, face powders, cuticle softeners, nail creams and lotions, oral hygiene products, mouthwashes, bath soaps, douches, feminine hygiene deodorants; shaving preparations, *e.g.,* aftershave lotions, skin care preparations, *e.g.,* cleansing, face and neck, body and hand, foot powders and sprays, moisturizing, night preparations, paste masks, skin fresheners; and suntan preparations, *e.g.,* gels, creams, and liquids.

Ammonia oxidizing microorganisms, *e.g.,* the *N. eutropha* may be associated with an aerosol, spray, or mist and these terms may be used interchangeably. An aerosol is typically a colloid of fine solid particles or fine liquid droplets, in a gas such as air. Aerosols may be created by placing the *N. eutropha* (and optionally carriers) in a vessel under pressure, and then opening a valve to release the contents. The container may be designed to only exert levels of pressure that are compatible with *N. eutropha* viability. For instance, the high pressure may be exerted for only a short time, and/or the pressure may be low enough not to impair viability. Examples of consumer uses of aerosols include for sunscreen, deodorant, perfume, hairspray, and insect repellant. The aerosol may be referred to as a spray or mist.

The compositions comprising ammonia oxidizing microorganisms, *e.g., N. eutropha* may also comprise one or more of a moisturizing agent, deodorizing agent, scent, colorant, insect repellant, cleansing agent, or UV-blocking agent.

Ammonia oxidizing microorganisms, *e.g., N. eutropha* may be associated with cloth, yarn, or thread. Articles of clothing such as, for example, shoes, shoe inserts, pajamas, sneakers, belts, hats, shirts, underwear, athletic garments, helmets, towels, gloves, socks, bandages, and the like, may also be treated with ammonia oxidizing bacteria, *e.g., N. eutropha.* Bedding, including sheets, pillows, pillow cases, and blankets may also be treated with ammonia oxidizing bacteria, *e.g., N. eutropha.* Areas of skin that cannot be washed for a period of time may also be contacted with ammonia oxidizing bacteria, *e.g., N. eutropha.* For example, skin enclosed in orthopedic casts which immobilize injured limbs during the healing process, and areas in proximity to injuries that must be kept dry for proper healing such as stitched wounds may benefit from contact with the ammonia oxidizing bacteria, *e.g., N. eutropha.*

The present disclosure provides a wearable article comprising ammonia oxidizing microorganisms as described herein. A wearable article may be a light article that can be closely associated with a user's body, in a way that does not impede ambulation. Examples of wearable articles include a wristwatch, wristband, headband, hair elastic, hair nets, shower caps, hats, hairpieces, and jewelry. The wearable article comprising an ammonia oxidizing bacteria, *e.g., N. eutropha* strain described herein may provide, *e.g.,* at a concentration that provides one or more of a treatment or prevention of a skin disorder, a treatment or prevention of a disease or condition associated with low nitrite levels, a treatment or prevention of body odor, a treatment to supply nitric oxide to a subject, or a treatment to inhibit microbial growth.

The ammonia oxidizing microorganisms, *e.g., N. eutropha* may be associated with a product intended to contact the hair, for example, a brush, comb, shampoo, conditioner, headband, hair elastic, hair nets, shower caps, hats, and hairpieces. Nitric oxide formed on the hair, away from the skin surface, may be captured in a hat, scarf or face mask and directed into inhaled air.

Articles contacting the surface of a human subject, such as a diaper, may be associated with ammonia oxidizing microorganisms, *e.g., N. eutropha.* Because diapers are designed to hold and contain urine and feces produced by incontinent individuals, the urea in urine and feces can be hydrolyzed by skin and fecal bacteria to form free ammonia which is irritating and may cause diaper rash. Incorporation of bacteria that metabolize urea into nitrite or nitrate, such as ammonia oxidizing bacteria, *e.g., N. eutropha,* may avoid the release of free ammonia and may release nitrite and ultimately NO which may aid in the maintenance of healthy skin for both children and incontinent adults. The release of nitric oxide in diapers may also have anti-microbial effects on disease causing organisms present in human feces. This effect may continue even after disposable diapers are disposed of as waste and may reduce the incidence of transmission of disease through contact with soiled disposable diapers.

The product comprising ammonia oxidizing microorganisms, *e.g., N. eutropha* may be packaged. The packaging may serve to compact the product or protect it from damage, dirt, or degradation. The packaging may comprise, *e.g.,* plastic, paper, cardboard, or wood. The packaging may be impermeable to bacteria. The packaging may be permeable to oxygen and/or carbon dioxide.

### Methods of Treatment with Ammonia Oxidizing Microorganisms

A subject may be treated via administration of ammonia oxidizing microorganisms, e.g., a preparation comprising ammonia oxidizing microorganisms. As used herein, treatment of a subject may comprise administering an ammonia oxidizing microorganism composition for a cosmetic or therapeutic result. For
instance, treatment may comprise treating or alleviating a condition, symptom, or side effect associated with a condition or achieving a desired cosmetic effect.

Subjects may include an animal, a mammal, a human, a non-human animal, a livestock animal, or a companion animal. The subject may be female or male. The subject may have various skin types. The subject may have various health-related profiles, including health history and/or genetic predispositions. The subject may generally have a normal microbiome, e.g., a physiological microbiome, or a disrupted microbiome. The subject may be characterized as one of the following ethnicity/race: Asian, black or African American, Hispanic or Latino, white, or multi-racial. The subject may be of an age of less than 1, or between 1-5, 5-10, 10-20, 20-30, 30-40, 40-50, 50-60, or over 60 years.

The ammonia oxidizing microorganisms that may be used to treat a subject include all the ammonia oxidizing microorganisms, *e.g., N. eutropha* compositions described in this application, *e.g.* a purified preparation of optimized ammonia oxidizing microorganisms, for instance strain D23.

The methods may be provided to administer, or deliver a therapeutic product or a cosmetic product. The methods may comprise administering or introducing a preparation comprising live ammonia oxidizing microorganisms to a subject. The preparation may be formulated to treat a target indication and/or formulated for a desired mode of delivery.

A preparation comprising live ammonia oxidizing microorganisms may be administered to a first tissue of a subject. The first tissue may be a deposit tissue. The first tissue may be a target tissue or a tissue other than a target tissue. The target tissue may be associated with a desired local or systemic effect. The target tissue may be associated with an indication, disorder, or condition to be treated.

Ammonia oxidizing microorganism preparations may be administered, for example to the skin, for a cosmetic or therapeutic effect. For instance, administration may provide a cosmetic treatment, benefit, or effect. Administration may provide for treatment or improvement of one or more of oily appearance, pore appearance, radiance, blotchiness, skin
tone evenness, visual smoothness, and tactile smoothness. A cosmetic appearance of a subject may be altered such as may result from improved skin health. Signs of aging may be reduced, delayed, or reversed. Administration may result in a qualitative improvement in skin and/or scalp condition and/or quality. Skin smoothness, hydration, tightness, and/or softness in a subject may be improved. The present disclosure also provides a method of reducing body odor.

Administration may provide a therapeutic treatment, benefit, or effect. The present disclosure provides a method of supplying nitrite and nitric oxide to a subject. The present disclosure provides various methods for the suppression, treatment, or prevention of diseases, disorders, infections, and conditions using ammonia oxidizing microorganisms. Ammonia oxidizing microorganisms may be used, for instance, to treat various diseases associated with low nitrite levels, skin diseases, and diseases caused by pathogenic bacteria. Administration may provide for a reduction in inflammation. Indeed, a local or systemic anti-inflammatory effect may be demonstrated. Inflammation may be downregulated. Microbial growth may be inhibited. Skin and overall health may be improved. Inadequate circulation may be augmented. Endothelial function may be promoted. A change in level of nitrite or NO at a target tissue or in circulation may be demonstrated. Administration, e.g., administration of an effective amount, may modulate, change, or alter a level of nitrite or NO at a target tissue or in circulation. Administration, e.g., administration of an effective amount, may result in an increased level of nitrite or NO at a target tissue or in circulation.

Administration of the compositions disclosed herein may provide transmucosal delivery and/or circulation, *e.g.* locally or systemically. Administration may provide that ammonia oxidizing microorganisms, products thereof, or byproducts thereof (e.g., nitrate, nitrite, NO, or CoQ8) penetrate a deposit or target tissue at least 10%, 20%, 30%, 40%, 50%, 60%, 70%, 80%, 90%, or 100%. 10%, 20%, 30%, 40%, 50%, 60%, 70%, 80%, 90%, or 100% of ammonia oxidizing microorganisms, products thereof, or byproducts thereof, may penetrate a deposit or target tissue or enter circulation upon administration of the compositions disclosed herein.

The preparations and methods of the present disclosure may provide for reducing an amount of undesirable microorganisms from an environment associated with a subject. The ammonia oxidizing microorganisms described herein may out-compete other organisms by, *e.g.,* consuming scarce nutrients, or generating byproducts that are harmful to other organisms, *e.g.,* changing a pH level that is not conducive to the undesirable organism's growth.

The present disclosure also provides a method of promoting wound healing, including of chronic wounds, such as in a patient that has an impaired healing ability, *e.g.,* a diabetic patient. A bandage including ammonia oxidizing microorganisms may optionally be applied to the wound.

It is appreciated that many modern degenerative diseases may be caused by a lack of NO species, and that AOM may be administered to supply those species, directly to a target tissue or via diffusion to a target tissue. Application of AOM may resolve long standing medical conditions. AOM may be applied to a subject to offset modern bathing practices, especially with anionic detergents which remove AOM from the external skin.

AOM may convert ammonia to nitrite, an anti-microbial compound, and nitric oxide, a well-documented signaling molecule in the inflammatory process.

The present disclosure provides, *inter alia,* a method of modulating a composition of a microbiome, e.g., modulating or changing the proportions of a microbiome in an environment, *e.g.,* a surface, *e.g.,* a surface of a subject. This may, in turn, exhibit a health-related benefit. The method may comprise administering a preparation comprising ammonia oxidizing microorganisms to a subject. The amount and frequency of administration, *e.g.,* application, may be sufficient to reduce a proportion of pathogenic microorganisms.

Application of ammonia oxidizing microorganisms to a subject, *e.g.,* a human subject may lead to unexpected changes in the microbiome. It may lead to increases in the proportion of normal commensal non-pathogenic species and reductions in the proportion of potentially pathogenic, pathogenic, or disease causing organisms.

An increase in the proportion of non-pathogenic bacteria may occur with a predetermined period of time, *e.g.,* in less than 1 day, 2 days, 3 days, 4 days, 5 days, 1 week, 2 weeks, 3 weeks, or 4 weeks, or in less than 1-3, 3-5, 5-7, 7-9, 5-10, 10-14, 12-18, 12-21, 21-28, 28-35, 35-42, 42-49, 49-56, 46-63, 63-70, 70-77, 77-84, 84-91 days.

A decrease in the proportion of pathogenic bacteria may occur with a pre-determined period of time, *e.g.,* in less than 1 day, 2 days, 3 days, 4 days, 5 days, 1 week, 2 weeks, 3 weeks, or 4 weeks, or in less than 1-3, 3-5, 5-7, 7-9, 5-10, 10-14, 12-18, 12-21, 21-28, 28-35, 35-42, 42-49, 49-56, 46-63, 63-70, 70-77, 77-84, 84-91 days.

A subject may be evaluated for need of treatment. A subject may be selected on the basis of the subject being in need of a treatment. The present disclosure may further provide obtaining a sample from a subject and analyzing the sample. Subjects may be evaluated before, during, and/or after treatment, such as at predetermined time intervals.

Administration may be performed before, during, or subsequent to occurrence of a health-related condition, or in response to a warning sign, trigger, or symptom thereof. A second amount of the preparation may be administered to the subject, e.g., a second dose.

The present disclosure provides combination therapies comprising ammonia oxidizing microorganisms, *e.g., a N. eutropha* and a second treatment, e.g. a therapeutic. For instance, the disclosure provides physical admixtures of the two (or more) therapies are physically admixed. The two (or more) therapies may be administered in combination as separate formulation. The second therapy may be, e.g., a pharmaceutical agent, surgery, diagnostic, or any other medical approach that treats, e.g., is approved to treat or commonly used to treat, the relevant disease, disorder, or a symptom of the relevant disease or disorder. The second treatment may be administered before or after the administration. The effective amount can be administered concurrently with the second treatment. The second treatment may be administered via the same or a different mode of delivery. The subject may have a therapeutic level of the second treatment upon administration of the preparation. The second treatment may provide an anti-inflammatory effect or be administered to reduce inflammation at the target site. The preparation may be administered concurrently or in conjunction with a product or byproduct of the ammonia oxidizing microorganisms, e.g., nitrite, nitrate, nitric oxide, CoQ8.
The preparation may be administered concurrently or in conjunction with a composition that promotes growth or metabolism of ammonia oxidizing microorganisms, promotes production of products or byproducts of ammonia oxidizing microorganisms, promotes urease activity, or has a synergistic effect with ammonia oxidizing microorganisms, e.g., ammonia, ammonium salts, urea, and urease.

The preparation may be administered with a microbiome cleansing preparation, for example a local or systemic antibiotic. The preparation may be administered after administration of a cleansing preparation or a bowel cleanse. The preparations may be administered pre- or post-surgical procedure, diagnostic procedure, or natural event, e.g., giving birth. The preparations may be administered before, during, or after deposit of an implantable or invasive device.

The preparation may be administered as an analgesic or prophylactic. The preparation may be self-administered. The administration of the preparation may be device-assisted.

The ammonia oxidizing microorganisms, *e.g.,* a preparation of ammonia oxidizing microorganisms, may be administered at a dose of about or greater than about 10³ - 10⁴ CFU, 10⁴ - 10⁵ CFU, 10⁵ - 10⁶ CFU, 10⁶ - 10⁷ CFU, 10⁷ - 10⁸ CFU, 10⁸ - 10⁹ CFU, 10⁹ - 10¹⁰ CFU, 10¹⁰ - 10¹¹ CFU, 10¹¹-10¹² CFU, 10¹²-10¹³ CFU, or 10¹³-10¹⁴ CFU per application, per day, per week, or per month. The ammonia oxidizing microorganisms may be administered at a dose of about 10⁹-10¹⁰ CFU, *e.g.,* about 1 x 10⁹ - 5 x 10⁹, 1 x 10⁹ - 3 x 10⁹, or 1 x 10⁹ - 10 x 10⁹ CFU per application or per day.

The ammonia oxidizing microorganisms may be administered in a volume of about 1-2, 2-5, 5-10, 10-15, 12-18, 15-20, 20-25, or 25-50 ml per dose. The solution may be at a concentration of about 10⁸-10⁹, 10⁹-10¹⁰, or 10¹⁰-10¹¹ CFU/ml. The ammonia oxidizing microorganisms may be administered as two 15 ml doses per day, where each dose is at a concentration of 10⁹ CFU/ml.

The ammonia oxidizing microorganisms may be administered once, twice, three, or four times per day. The ammonia oxidizing microorganisms may be administered once, twice, three, four, five, or six times per week.
The ammonia oxidizing microorganisms may be administered shortly after bathing. The ammonia oxidizing microorganisms may be administered shortly before sleep.

The ammonia oxidizing microorganisms may be administered for about 1-3, 3-5, 5-7, 7-9, 5-10, 10-14, 12-18, 12-21, 21-28, 28-35, 35-42, 42-49, 49-56, 46-63, 63-70, 70-77, 77-84, 84-91 days, *e.g.,* for about 1 month, for about 2 months, for about 3 months. The ammonia oxidizing microorganisms may be administered for an indefinite period of time, e.g., greater than one year, greater than 5 years, greater than 10 years, greater than 15 years, greater than 30 years, greater than 50 years, greater than 75 years.

### Administration of Ammonia Oxidizing Microorganisms to the Intranasal System

The formulations (*e.g*., preparations or compositions) described herein may include those suitable for intranasal delivery, *e.g.,* topical administration, inhalation, and via olfactory transfer. Ammonia oxidizing microorganism preparations may be administered to the nasal cavity for cosmetic or therapeutic purposes. For instance, compositions include those formulated for cosmetic or therapeutic use.

Any ammonia oxidizing microorganism (AOM) can be used, provided it can survive in the nasal passages. Generally, the nasal passages may be at a temperature of about 38° and contain fluids with the osmotic strength of nasal secretions.

Compositions disclosed herein may comprise AOM suspended or dispersed in a fluid which is non-irritating to the nasal passages, throat, or lungs, for example, saline, air, buffered saline, or AOM storage buffer, as previously disclosed herein. Lyophilized AOM, dispersed in air, may also be used. Isotonic saline (e.g., about 0.9% NaCl in distilled water), diluted sea water, and undiluted sea water are common nasal irrigants and may be used as vehicle for the compositions disclosed herein.

Compositions disclosed herein may comprise a low nitrite carrier fluid. Nitrite can be added as a preservative, buffer, or to supplement the normal nitrite level of nasal secretions. When the nasal epithelium generates NO from nNOS, a significant portion of NO may be captured as nitrite in the nasal mucous layer. AOM may generate nitrite as well as NO, such that nitrite may be self-generated in the composition by adding a source of ammonia to the living culture of AOM.

The intranasal formulations (*e.g*., preparations or compositions) may conveniently be presented in unit dosage form and may be prepared by any of the methods known in the art of pharmacy or cosmetology. Typically, methods include the step of bringing the active ingredient (*e.g*., ammonia oxidizing microorganism) into association with a pharmaceutical carrier which constitutes one or more accessory ingredients. In general, the pharmaceutical or cosmetic formulations are prepared by uniformly and intimately bringing into association the active ingredient with liquid carriers or finely divided solid carriers or both and then, if necessary, shaping the product into the desired formulation.

Intranasal formulations may be presented as discrete units, each containing a predetermined amount of the active ingredient as a solution or suspension in an aqueous or non-aqueous liquid, as a powder or granules, or as an oil-in-water or water-in-oil liquid emulsion. Various pharmaceutically acceptable carriers and their formulations are described in standard formulation treatises, e.g., Remington's Pharmaceutical Sciences by E.W. Martin. See also Wang, Y.J. and Hanson, M.A., Journal of Parenteral Science and Technology, Technical Report No. 10, Supp. 42:2 S, 1988; Aulton, M. and Taylor, K., Aulton's Pharmaceutics: The Design and Manufacture of Medicines, 5th Edition, 2017; Antoine, A., Gupta M.R., and Stagner, W.C., Integrated Pharmaceutics: Applied Preformulation, Product Design, and Regulatory Science, 2013; and Williams, R., Taft, D., and McConville, J., Advanced Drug Formulation Design to Optimize Therapeutic Outcomes, Drugs and the Pharmaceutical Sciences, vol. 172, 2007.

Compositions disclosed herein may be prepared in intranasal dosage formulations. Ammonia oxidizing microorganisms can administered intranasally for cosmetic or therapeutic purposes. Intranasal formulations are generally intended for absorption through the various epithelia and mucosa of the nasal cavity. For instance, compositions may be prepared as tablets, capsules, solutions, suspensions, emulsions, or suppositories. Each of the dosage forms may be formulated to comprise one or more carrier or excipient, as described in more detail below. Generally, liquid dispersion forms, *e.g.,* intranasal dispersion forms, may comprise solutions, suspensions, or emulsions of the active agent in a vehicle.

Compositions prepared for intranasal administration may be formulated as a solution, suspension, emulsion, ointment, bougie, powder. gel, hydrogel, or liquid. Typically, intranasal liquids or solutions may be aqueous solutions, *e.g.,* an aqueous dispersion of the active agent. The intranasal formulation may be a drop, spray, aerosol, or mist. Intranasal ointments may comprise anhydrous dispersions of the active agent, *e.g.,* in a mineral oil-white petroleum base. Intranasal gels may comprise a polymer, *e.g.,* poloxamers, xanthan gum, gellan gum, locust bean gum, and carrageenan. Nasal ointments and gels may provide for a longer residence time than, for example, aqueous solutions. The longer residence time may be provided by bioadhesion, and may further allow for a reduced dosing interval. Intranasal emulsions may comprise microspheres, microcapsules, nanoparticles, nanocapsules, micelles, liposomes, niosomes, dendrimers, or cyclodextrin complexes. Intranasal powders may be solid particles, *e.g.,* having a diameter of between about 5 µm to about 20 µm that remain in the nasal cavity when administered. Intranasal bougies may be formulated as a suppository, *e.g.,* having a gel or gelato-glycerin base. Any of the aforementioned formulations may be freeze-dried or lyophilized for ease of administration. A composition may be delivered directly to a target tissue, e.g., a target nasal cavity tissue. The target nasal cavity tissue may be proximate an inlet of the nasal cavity of a subject.

Ammonia oxidizing compositions disclosed herein may comprise an effective amount of AOMs, for example, to increase mucus thickness in at least a portion of the nasal cavity, to colonize a target tissue of the nasal cavity of the subject, to induce ischemic preconditioning anti-triggering in the subject, to treat neurogenic inflammation or a symptom of neurogenic inflammation, to treat a neurological disorder or a symptom of a neurological disorder, to treat a nasal or sinus disorder or a symptom of a nasal or sinus disorder, to decongest, to decrease sinus pressure, or to change or modulate an inflammatory response in the subject. The compositions may be administered to the nasal cavity of the subject. The compositions may be administered after a nasal cavity cleanse or antibiotic treatment. The nasal cavity may be substantially cleared when the preparation is administered, *e.g.,* by cleanse or decongestant treatment. Water or buffer may be administered to the subject after administration of the ammonia oxidizing microorganism composition. Several hours may be waited prior to administering water or buffer to the subject or cleansing the nasal cavity of the subject. AOM may not be administered during a stress state for the subject.

Such compositions may be formulated for topical application, inhalation, olfactory transfer administration, or device-assisted application. Topical application formulations may include, *e.g.,* bougie, powder, gel, drop, spray, aerosol, and mist. Inhalation formulations may include, e.g., powder, fine spray, aerosol, or mist. Instillation formulations may include, e.g., drop, wash, gel, or hydrogel. Olfactory transfer formulations may be specifically formulated to contact the olfactory region of the nasal cavity, diffusing the active ingredient through the olfactory pathway and eventually through the blood brain barrier (BBB) into the central nervous system (CNS). Application or administration may be achieved by inserting the formulation in the nasal cavity, either with or without the assistance of a device. Device-assisted application may include, for example, delivery via an applicator or an insertable applicator, catheter, inhaler, nebulizer, or delivery in conjunction with an endoscope or ultrasound. Suitable applicators include liquid formulation bulbs, squeeze bottles, metered-dose sprays, spray pumps, neti pots, and airless spray pumps and solid formulation metered-dose applicators, *e.g.,* for delivery of powders, and insertable applicators.

The time of onset of action for the formulations disclosed herein may be dependent on the formulation and may range from seconds to minutes to hours. Suppositories, solutions, and suspensions may provide action within minutes or hours. Powders, granules, tablets, and capsules may provide action within minutes to hours. Modified release formulations may provide action within minutes to hours. The release time for the formulations disclosed herein may be dependent on the formulation and may range from minutes to hours to days. For example, the dosage forms may be formulated to provide fast-release within minutes or extended release within hours. Certain dosage forms may provide extended release within days or months.

Intranasal administration may provide certain benefits for the AOM compositions disclosed herein. Specifically, intranasal administration is painless, easy to administer (*e.g*., may be self-administered), does not require invasive procedures, avoids first pass metabolism (*e.g*., through the gastrointestinal system), does not require sterility of the formulation, and allows direct delivery to the CNS through the olfactory pathway. The rich vasculature of the nasal cavity may provide direct route into the bloodstream for systemic effects. However, active agents must be formulated to cross the mucous membrane to reach the nasal cavity vasculature. Furthermore, the rapid rate of mucociliary transit in the nasal cavity may be the major barrier to intranasal drug absorption. The preparation may be formulated to be
compatible with the mucous membrane of the nasal cavity of the subject. The preparation may be formulated for transmucosal delivery, *e.g.,* local or systemically.

Ammonia oxidizing microorganism compositions disclosed herein may comprise absorption enhancers, *e*.*g*., bile salts or fusidate derivatives, to increase absorption of the substances across the nasal mucosa layer. Suitable bile salts include cholate, sodium taurocholate, sodium glycocholate, and sodium deoxycholate. Bile salts may promote absorption by increasing permeability of the membrane, inhibiting proteolytic enzymes, forming aqueous pore pathways in the membrane, or solubilizing the drug in aqueous solution as a result of their surfactant properties. Fusidate derivatives may employ similar mechanisms to enhance absorption of the active agent. Suitable fusidate derivatives include sodium fusidate, sodium dihydrotaurofusidate, and sodium salts of fusidic acids.

Compositions disclosed herein may further comprise bioadhesive agents, e.g., chitosan polymers. Bioadhesive agents may enhance absorption by increasing the permeability of the mucosal layer. Cyclodextrins may further be included in the compositions disclosed herein to form inclusion complexes with the active ingredient. The inclusion complexes may be formulated to improve absorption of the drug. Suitable cyclodextrin absorption promoters include dimethyl-β-cyclodextrin phospholipids (DMβC), *e.g.,* phosphotidylcholines, *e.g.,* lysophosphatidylcholine and didecanoyl-L-phosphatidylcholine, optionally, in combination with degradable starch microspheres. Other compounds that may be included in the formulations disclosed herein to serve as nasal mucosa adhesion and penetration enhancers include menthol, ammonium glycyrrhizinate and glycyrrhetinic acid, aminated gelatin, enzyme inhibitors (*e*.*g*., aminoboronic acid derivatives, amastatin), microparticle resins, alkyl maltosides, and alkyl sucrose esters (*e*.*g*., glycofurol, polyacrylic acid gel, alginic acid, microcrystalline cellulose).

Bougies may be solid dosage forms intended for introduction into the nasal cavity. The bougie may melt, releasing the active agent, once introduced into the nasal cavity. The rate of delivery of the active agent may be influenced by selection of pharmaceutically acceptable carrier or base. Suitable bases include fatty bases and water bases. Suitable fatty base formulations may comprise theobroma oil (cocoa butter), spermaceti (beeswax), synthetic triglycerides or triglycerides from hydrogenated vegetable oils, palm, palm kernel, or coconut oils. Suitable water base formulations may comprise glycerinated gelatin or polyethylene glycol polymers. Bougies may be prepared in a variety of shapes, *e.g.,* cone shaped. Bougies may be formulated to include one or more of fillers, binders, bulking agents, diluents, disintegrants, lubricants, anti-adherents and anti-sticking agents, glidants and flow agents, wetting agents, solubilizing agents, drug-release modifiers, stabilizers, and colorants.

Ointments, foams, and gels may generally be formulated to be more viscous than aqueous solutions and provide for a longer residence time within the nasal cavity. Such viscous liquid formulations may comprise a gel or gelling agent. For instance, a gelling agent may be a thermoreversible gel. A thermoreversible gel may be a liquid at lower or room temperature and turn to gel once inserted into the nasal cavity. The gel or gelling agent may allow easier administration and positioning of the dosage form. For instance, the gel or gelling agent may prevent the dosage form from leaking out of the body cavity. Thermoreversible polymers include poloxamer. Mucoadhesive polymers include sodium alginate. Gels or gelling agents may further comprise a solubility enhancer, for example, hydroxypropyl-betalcyclodextrin. Gel formulations may include, but are not limited to agar, silica, polyacrylic acid (for example Carbopol^{®}), carboxymethyl cellulose, starch, guar gum, alginate or chitosan.

Solutions containing the compositions disclosed herein may be formulated as, *e.g.,* drops, a spray, an aerosol, a mist, or a wash. Generally, these solutions may be aqueous solutions comprising the active agent. The solutions may be administered to reach a deep nasal cavity tissue, *e.g.,* the olfactory region, or a superficial nasal cavity tissue, *e.g.,* the septal wall. Solutions may be administered with an applicator, e.g., squeeze bottle, metered-dose spray, spray pump, airless spray pump, neti pot, and bulb. Certain antiseptics used during nasal washes may disturb the natural balance of microorganisms in the nasal cavity, causing infections. Such washes may be administered in combination with the ammonia oxidizing microorganism compositions disclosed herein to restore the balance of microorganisms. Furthermore, the ammonia oxidizing microorganism compositions disclosed herein may be administered in a solution as a primary or secondary therapy, for example, in combination with one or more additional treatments.

Intranasal powders may be solid particles. The powder particles may have a diameter of less than about 5 µm, less than about 10 µm, less than about 15 µm, less than about 20 µm, less than about 25 µm, less than about 30 µm, less than about 50 µm, or less than about 100 µm. The powder particles may have a diameter of between about 1 µm and about 50 µm, between about 1 µm and about 30 µm, or between about 5 µm and about 20 µm. The solid powder particles may be formulated to remain in the nasal cavity when administered or be inhaled into the respiratory system. The solid particles may be prepared, *e*.*g*., by freeze-drying or lyophilizing compositions comprising AOM.

Exemplary compositions may include one or more excipients, for example, absorption and penetration enhancers, analgesics, local analgesics, antifungal agents, anti-inflammatory agents, steroids and corticosteroids, thermoreversible gels, preservatives, antioxidants, buffers, chelating agents, ion exchange agents, solubilizing agents, suspending agents, thickeners, surfactants, wetting agents, tonicity-adjusting agents, and a vehicle for proper drug delivery. Absorption and penetration enhancers may improve the ability of the active agent to be absorbed by a number of different mechanisms. Analgesics and local analgesics may be used to relieve pain and/or decrease subject discomfort. Steroids and corticosteroids may help reduce inflammation. Thermoreversible gels may improve positioning and retention time of the active agent. Antioxidants may reduce the oxidative degradation of the active agent. Buffers may maintain a desired pH of the composition and/or enhance solubility or stability of the composition. Chelating agents may include complex trace metals that catalyze oxidation reactions of the composition. Ion exchange agents may control the release of active agent by ion exchange mechanisms. Solubilizing agents may increase the solubility of the active agent or another excipient. Suspending agents and thickeners may increase the viscosity or density of the composition to increase the active agent's retention time and residence time in the gastrointestinal system, for example the oral or rectal cavity. Surfactants, including cationic, anionic, and non-ionic surfactants, and wetting agents may act to wet insoluble hydrophobic active agent or other excipients. Tonicity-adjusting agents may provide an isotonic solution with urogenital fluids. Vehicle, for example water or fatty base, may provide bulk for proper active agent delivery.

Excipients that can be included are, for instance, proteins, such as human serum albumin or plasma preparations. If desired, the pharmaceutical composition may also contain minor amounts of non-toxic auxiliary substances, such as wetting or emulsifying agents, preservatives, and pH buffering agents and the like, for example sodium acetate or sorbitan monolaurate. Excipients, e.g., a pharmaceutically acceptable excipient or a cosmetically acceptable excipient, may comprise an anti-adherent, binder, coat, disintegrant, filler, flavor, color, lubricant, glidant, sorbent, preservative, or sweetener. In The preparation may be substantially free of excipients.

The preparation may be substantially free of one or more of the compounds or substances listed in the disclosure.

The ammonia oxidizing microorganism compositions can, for example, be administered in a form suitable for immediate release or extended release. Topical formulations for immediate release may include, e.g., solutions, suspensions, emulsions, foams, gels, and ointments. Topical formulations may be formulated for immediate release to avoid complications from clearance by bodily fluids or ciliary cells. Intranasal delivery formulations may be formulated to experience a phase change upon coming into contact with bodily fluids within the nasal cavity and release the active agent. For instance, bougies and solutions may be formulated for immediate release to avoid challenges caused by dosage form expulsion and low adhesion to the nasal cavity mucosa. The nasal mucosa may allow for quick active agent absorption of compositions formulated for transmucosal delivery, while rich localized vasculature enables easy update to systemic circulation.

Controlled release nasal formulations may be prepared as liquid dispersion or solid dispersion forms. Suitable examples of sustained-release systems include suitable gelating or polymeric materials (polymer based cores or coating membranes), for example semi-permeable polymer matrices in the form of shaped articles, e.g., polymer gels, or microcapsules, suitable hydrophobic materials, for example as an emulsion in an acceptable oil, or ion exchange resins. The pharmaceutical compositions may be in the form of particles comprising one or more of biodegradable polymers, polysaccharide jellifying and/or bioadhesive polymers, or amphiphilic polymers. These compositions exhibit certain biocompatibility features which allow a controlled release of an active substance.

Controlled release nasal formulations may be formulated with one or more mucoadhesive agent, *e*.*g*., mucoadhesive gel or dry mucoadhesive. The mucoadhesive agent may aid in attachment to the nasal cavity tissue, *e*.*g*., nasal mucous membrane. The mucoadhesive agent may enhance dosage form positioning within the nasal cavity or may remain present when part of the solid dosage form melts or disintegrates. For instance, a solid dosage form may be formulated to dissolve rapidly when in contact with bodily fluid and turn to a mucoadhesive viscous solution that attaches to the nasal mucosa and is gradually washed out without requiring removal. Mucoadhesive agents may attach to the nasal mucosa, permitting slow release of the drug directly to the nasal vasculature over a long period of time.

The preparations may be one or more of: substantially odorless, colorless, not associated with substantial side effects, non-toxic, well-tolerated, have no adverse effects if released into the environment, no risk of fostering antibiotic resistance, and have a physiology such that it can interact positively with various human gut microbiomes under normal and disease states.

The ammonia oxidizing microorganism compositions can, for example, be administered in form suitable to provide local treatment or systemic treatment. Local effects may be achieved, for example, by rapid absorption through the tissues of the intranasal system, and systemic effects may be achieved, for example, by drug absorption through various epithelia and mucosa of the nasal cavity tissues. Compositions disclosed herein may be administered to treat a local inflammatory disease, a symptom of a local or systemic inflammatory disease, or a side effect caused by a local or systemic inflammatory disease. The compositions may treat a primary inflammatory disease or symptom, or a secondary inflammatory disease or symptom. The inflammatory condition may be associated with a medical event, *e.g*., an injury, cancer, or an infection. The inflammatory disease may follow an injury, *e*.*g*., spinal cord injury, head trauma, or a brain injury. The inflammatory condition may be associated with a nasal or sinus condition or disorder or a symptom or side effect of a nasal or sinus condition or disorder. Suitable examples of local nasal or sinus conditions or disorders that may be treated with compositions disclosed herein may be allergic rhinitis. The nasal or sinus condition or disorder may be associated with an allergen, a bacterial infection, or a viral infection, *e*.*g*., coronavirus, rhinovirus, meningitis, or influenza. The nasal or sinus condition or disorder may be characterized by congestion or sinus pressure. The nasal or sinus condition or disorder is characterized or accompanied by congestion, sinus pressure, sneezing, sinusitis, asthma, or discomfort.

Compositions disclosed herein may be administered to treat a neurological disorder, a symptom, or side effect of a neurological disorder. The neurological disorder may be associated with neurogenic inflammation. The neurogenic inflammation may be associated with or accompanied by headache, neuropathy (*e*.*g*., diabetic neuropathy, peripheral neuropathy, Lewy body neuropathy), epilepsy, systemic sclerosis, multiple sclerosis, amyotrophic lateral sclerosis, Alzheimer's Disease, Parkinson's Disease, white matter hyperintensities, diabetic retinopathy, anxiety, post-traumatic stress disorder, chronic fatigue syndrome, fibromyalgia, depression, insomnia, arthritis, rheumatoid arthritis, allergic rhinitis, dilative cardiomyopathy, atherosclerosis, cardioprotection, heart failure, hypertension (e.g., pulmonary hypertension, gestational hypertension, portal hypertension), eclampsia, pre-eclampsia, capillary rarefaction, peripheral vasculopathy, gestational diabetes, type 2 diabetes, obesity, metabolic syndrome, kidney failure, liver failure, pancreatitis, or hepatitis. The compositions disclosed herein may be administered to treat a degenerative neurological disorder, a genetic neurological disorder, a psychological neurological disorder, or a symptom or side effect thereof. Compositions disclosed herein may be administered to treat headaches or hypertension.

In the claimed invention, the preparation is for use in a method of treating allergic rhinitis in a subject.

An intranasal or nervous system state of a subject may be impacted, *e*.*g*., a composition of a intranasal system or CNS microbiome may be changed, altered, or modulated, such as by changing proportions of microorganisms therein, such as in the nasal cavity or CNS. A systemic composition of a microbiome may be changed, altered, or modulated, *e.g*., by changing proportions of microorganisms in the gastrointestinal system, endocrine system, other system, or systemically.

Ammonia oxidizing microorganism compositions can be administered in a form suitable to treat certain infections and inflammatory disorders, e.g., bacterial infections, fungal infections, viral infections, itching, local inflammation, and wound healing. For instance, ammonia oxidizing microorganism compositions may be administered to treat inflammation associated with a surgical or diagnostic procedure, dental treatment, catheter-based transfers (e.g., matter transfers in, out, or in between two locations within the body), or generally inflammation related to any foreign body introduced into the intranasal system. Administration of a composition disclosed herein may precede or follow a medical or surgical procedure, *e*.*g*., catheterization, endoscopy, intubation, (*e*.*g*., nasogastric intubation), placement of a nasal cannula, or a dental procedure. Compositions disclosed herein may be administered to treat localized symptoms or side effects of systemic conditions or disorders. For instance, ammonia oxidizing microorganism compositions may be administered to treat congestion, sinus pressure, sneezing, discomfort, sinusitis, asthma, or headaches associated with a systemic or remote condition or disorder.

Examples of systemic conditions that may be treated with compositions disclosed herein include headaches, cardiovascular diseases, connective tissue disorders, inflammation, immune responses and autoimmune disorders, liver diseases, infections, neurological diseases, psychiatric disorders, nitric oxide disorders, urea cycle disorders, congestion, vasodilation disorders, skin diseases, wound healing, bowel disorders, reactions to insect bites, ophthalmic disorders, and certain viral, bacterial, and fungal infections. For instance, systemic conditions that may be treated with compositions disclosed herein include cardiovascular diseases such as cardioprotection, heart failure, hypertension, pulmonary arterial hypertension; immune responses and autoimmune disorders such as alopecia and vitiligo; liver diseases such as non-alcoholic fatty liver disease (NAFLD), non-alcoholic steatohepatitis (NASH); neurological diseases and psychological disorders such as depression, insomnia, and diabetic neuropathy; nitric oxide disorders such as erectile dysfunction; wound healing, *e*.*g*., from bed sores and nursing home care, burns, diabetic ulcers *e.g.,* foot ulcer, venous leg ulcer, biofilm, and mouth sores; skin diseases and disorders such as hyperhydrosis, pruritus, helomas, and subtypes of helomas; ophthalmic disorders such as blepharitis, dry eye, macular degeneration, and glaucoma; bowel disorders such as gluten sensitivity, irritable/inflammatory bowel disease, Crohn's disease, colitis, and necrotizing enterocolitis; and vasodilation disorders such as Renaud's disease, thermoregulation, and migraines. Certain viral, bacterial, and fungal infections may be treated with formulations disclosed herein, including infections caused by human papillomavirus (HPV), yeast infections, tinea versicolor, tinea unguium, tinea pedis/fungus, tinea cruris, jock itch, onychomycosis, dandruff, athlete's foot, sinusitis, otitis media, Methicillin-resistant *Staphylococcus aureus* (MRSA), staph, and bacterial vaginosis. Additional systemic conditions that may be treated with compositions disclosed herein include systemic inflammation, such as eczema, *e.g.,* adult and pediatric eczema, hives, idiopathic uriticaria, lichen planus, insect bites including allergic reactions to insect bites, *e*.*g*., mosquito and demodex folliculorum mite, reactions to poison ivy, itchiness, keratosis pilaris, laryngitis, pemphigus, psoriasis, rosacea, and of nerioral folliculitis suhtypes folliculitis, hidradenitis supportiva, dermatitis, lupus rash, seborrheic dermatitis, *e.g.,* adult and infantile seborrheic dermatitis, acne, *e.g.,* adolescent acne, adult acne, and cystic acne, diaper rash, occupational hand dermatitis, sunburn, and dermatomyositis. Additionally, compositions disclosed herein may be delivered or applied to treat certain cosmetic indications, including but not limited to, contact dermatitis, diaper odor, *e.g.,* adult and pediatric, body odor, feminine odor, flaking, nail hardness, body odor, oily skin, razor burn, skin appearance, skit blotchiness, skin hydration, and sun spots. Compositions disclosed herein may be applied as a bug repellant or an antimicrobial agent.

Compositions disclosed herein may further be formulated as combination therapies. For instance, bougies may comprise distinct sections formulated for combination therapy. Initial and subsequent therapeutic treatments may be provided in a single dosage form, prepared in individual dosage forms, administered concurrently, or administered separately. Individual dosage forms may be administered via the same mode of administration, *e*.*g*., through the intranasal system, or via an alternate mode of administration, *e*.*g*., orally, enterally, topically, ocularly, via the auditory system, via the urogenital system, via the respiratory system, or via injection. For instance, combination therapies may comprise ammonia oxidizing microorganisms for treatment of an inflammatory disease or condition. Individual dosage forms may be administered by a surgical or diagnostic procedure. Individual dosage forms may be administered in combination with a surgical or diagnostic procedure. Ammonia oxidizing microorganism compositions, for example prepared for intranasal administration, are formulated for combination therapy with an anti-inflammatory. Generally, compositions disclosed herein may be formulated for combination therapy with a drug or compound approved or commonly used to treat a disease, disorder, condition, symptom thereof, or side-effect thereof, for example a neurological, nasal, or sinus disease, disorder, condition, symptom thereof, or side-effect thereof. Preparations may be formulated for administration in combination with a nasal cleanse or antibiotic to "plow the field" and allow for ammonia oxidizing microorganism colonization. Ammonia oxidizing microorganism compositions disclosed herein may be administered in combination with a therapeutic treatment for one or more of headache, neuropathy (*e*.*g*., diabetic neuropathy, peripheral neuropathy, Lewy body neuropathy), epilepsy, systemic sclerosis, multiple sclerosis, amyotrophic lateral sclerosis, Alzheimer's Disease, Parkinson's Disease, white matter
hyperintensities, diabetic retinopathy, anxiety, post-traumatic stress disorder, chronic fatigue syndrome, fibromyalgia, depression, insomnia, arthritis, rheumatoid arthritis, allergic rhinitis, dilative cardiomyopathy, atherosclerosis, cardioprotection, heart failure, hypertension (*e*.*g*., pulmonary hypertension, gestational hypertension, portal hypertension), eclampsia, pre-eclampsia, capillary rarefaction, peripheral vasculopathy, gestational diabetes, type 2 diabetes, obesity, metabolic syndrome, kidney failure, liver failure, pancreatitis, or hepatitis. Compositions disclosed herein may additionally or alternately be administered in combination with a therapeutic treatment for allergic rhinitis, a bacterial infection, a viral infection, *e*.*g*., coronavirus, rhinovirus, meningitis, or influenza, or a symptom or side effect thereof, *e.g.,* congestion, sinus pressure, sneezing, sinusitis, asthma, discomfort, or headache.

Preparations for administration to the intranasal system may be formulated for targeted delivery to a specific deposit tissue or target tissue. The preparation or product of the preparation may be delivered locally or systemically, *e*.*g*., at a deposit or target tissue or in circulation. The deposit tissue or target tissue may be associated with a desired systemic effect.

The intranasal route can be useful in pediatric and geriatric groups and patients who are unable to take oral medications due to nausea, vomiting, and unconsciousness. Intranasal administration may be utilized to deliver drugs for preventing pre- and post-operative infections and treating inflammation. Preparations for intranasal administration may be formulated for targeted delivery to a specific nasal cavity tissue. Intranasal deposit or target tissues include the nasal cavity, septal wall, nasal valve, nostril, nasopharanyx, vestibular area, turbinate (inferior, middle, superior), meatus (inferior, middle, superior), concha (inferior, middle, superior), maxillary sinus, sphenoidal sinus, sphenoethmoidal recess, ethmoidal bulla, semi-lunar hiatus, nasolacrimal duct, frontonasal duct, or olfactory region of the subject. Compositions may be specially formulated to bypass the mucous membrane of the nasal cavity and deliver the active agent to another target tissue, e.g., systemically. Generally, the intranasal route may provide for systemic effects through the large available surface area and blood supply of nasal cavity tissues. Compositions may be specially formulated to contact the olfactory region and deliver the active agent to the CNS through the BBB via olfactory transfer, as previously described. Olfactory transfer may be beneficial for compositions formulated to treat a neurological disorder and/or transfer AOM to the CNS.

Administration of a preparation comprising AOM may anti-trigger ischemic preconditioning or modulate an ATP level in the subject. In the nasal passages, ammonium in the extravesicular space may provide substrate to the AOM, so that the AOM produce NO and nitrite which may anti-trigger an ischemic preconditioned state. While not wishing to be bound by any particular theory, it is believed that one mechanism by which intranasal AOM may act as an anti-trigger of ischemic preconditioning relates to how ischemia, hypoxia, oxidative stress, or ATP depletion activate the ATP sensitive potassium channels. The channels open when ATP levels inside the affected cell drop, releasing potassium ions into the extracellular space. The release of potassium ions propagates a wave of depolarization, whereby adjacent cells experience a similar drop in ATP levels, propagating the state of ischemic preconditioning across the relevant tissue compartment (*e*.*g*., the brain). During this wave, AOM convert ammonia released into the extracellular space by the potassium channels into NO and nitrite, which may serve to quench the ongoing spreading state of ischemic preconditioning, thereby anti-triggering the response.

While not wishing to be bound by any particular theory, it is believed that prolonged states of ischemic preconditioning contribute to the adverse health effects exhibited in by individuals with chronic stress. Specifically, it is believed that the triggering of ischemic preconditioning overlaps with the triggering of the trigemino-cardiac reflex. The trigemino-cardiac reflex may signal a reduction in oxygen consumption and the activation of oxygen conserving pathways. Thus, the physiology described herein can further contribute to long-term degenerative effects by signaling oxidative stress mechanisms, *e*.*g*., unwanted reactive oxygen species, *e*.*g*., super oxide. The compositions disclosed herein may alleviate the effects of chronic stress and oxidative stress by anti-triggering the ischemic preconditioned state.

During an acute stress event, physiology may trigger inflammation to try and preserve organism viability. Generally, following a successful resolution of an acute stress event, physiology triggers anti-inflammation to exit the ischemic preconditioning state and return the body to a state of rest. While not wishing to be bound by a particular theory, it is believed that chronic and oxidative stress, along with the resultant prolonged states of ischemic preconditioning, contribute to disorders associated with neurogenic inflammation. Disorders that have been found to be associated with neurogenic inflammation include neurological disorders, symptoms and side effects thereof, *e.g.,* headache, neuropathy (e.g., diabetic neuropathy, peripheral neuropathy, Lewy body neuropathy), epilepsy, systemic sclerosis, multiple sclerosis, amyotrophic lateral sclerosis, Alzheimer's Disease, Parkinson's Disease, white matter hyperintensities, diabetic retinopathy, anxiety, post-traumatic stress disorder, chronic fatigue syndrome, fibromyalgia, depression, insomnia, arthritis, rheumatoid arthritis, allergic rhinitis, dilative cardiomyopathy, atherosclerosis, cardioprotection, heart failure, hypertension (e.g., pulmonary hypertension, gestational hypertension, portal hypertension), eclampsia, pre-eclampsia, capillary rarefaction, peripheral vasculopathy, gestational diabetes, type 2 diabetes, obesity, metabolic syndrome, kidney failure, liver failure, pancreatitis, or hepatitis.

Without wishing to be bound to any particular theory, as previously described, during inflammation or hypoxic triggering of ischemic preconditioning, cells may release ATP, ADP, and AMP. The ATP, ADP, and AMP are activated upon by phosphatases which remove the phosphates leaving adenosine. Adenosine may be deaminated by deaminase enzymes on the cell surface, producing another source or ammonia for AOM to use to generate NO and nitrite. As previously described, the AOM, NO, and nitrite may contribute to anti-triggering of a state of ischemic precondition, thus reducing a state of inflammation, including neurogenic inflammation, in the subject.

Furthermore, without wishing to be bound by particular theory, it is believed that NO may be the normal regulator of blood flow, mitochondria biogenesis, ATP status, and allocation of resources to immediate consumption or to repair. Neurogenic inflammation may trigger systemic inflammation by way of response to an immune system signal. The nervous system may detect a locally triggered inflammation (primary inflammation) and trigger inflammation (secondary inflammation) in a region remote from the primary inflammation. Systemic inflammatory conditions, e.g., triggered neurogenically, may be associated with adverse liver effects such as portal hypertension, primary sclerosing cholangitis, non-alcoholic steatohepatitis, non-infectious hepatitis and also multiple systemic inflammatory conditions such as asthma, Multiple sclerosis, chronic renal disease, psoriasis, pericarditis, and arthritis. Similarly, diabetes, stroke, and heart disease may be associated with chronic systemic inflammatory disorders, e.g., inflammatory disorders in the gastrointestinal system. Expanded blood volume and/or hyperdynamic circulatory state, such as in the splanchnic system may result. Decreased renal blood flow may be caused which may lead to kidney failure. Prevention of systemic inflammation by colonization with AOM in accordance with various instances may also reduce the incidences of other systemic inflammatory conditions.

Children affected by abuse, trauma, or low socioeconomic status may be at risk for adverse immune system effects thereof. In children, administration of AOM to the intranasal system, and the associated reduction of oxidative stress, can mitigate neurodevelopmental problems associated with the resultant neurogenic inflammation.

### Use of Microbiome Compatible Products with Administration of Ammonia Oxidizing Microorganisms

Microbiome compatible products may be used in conjunction with the preparations and methods disclosed herein. Various products may be considered to be "biome-friendly" or "biome-compatible." Examples of biome-friendly products are disclosed in International (PCT) Patent Application Publication No. WO2017/004534 (International (PCT) Patent Application Serial No. PCT/US/2016/040723 as filed on July 1, 2016). Some biome-friendly products may be cosmetic or therapeutic in nature. Biome-friendly products may be used in combination with microorganisms, e.g., non-pathogenic microorganisms, e.g., ammonia oxidizing microorganisms, which may in turn be used in the form of a preparation or composition to be applied to a subject. Ammonia oxidizing compositions disclosed herein may be administered for a cosmetic or therapeutic indication in conjunction with a biome-friendly or biome-compatible product.

A preparation, composition, formulation or product comprising ammonia oxidizing microorganisms, e.g., for cosmetic or therapeutic use, may itself be considered biome-friendly. A preparation comprising ammonia oxidizing microorganisms may be used in conjunction with a biome-friendly product. A preparation comprising ammonia oxidizing microorganisms may be mixed with a biome-friendly product or otherwise administered concurrently. A preparation comprising ammonia oxidizing microorganisms may be distinct or separate from a biome-friendly product although potentially used in conjunction therewith. A biome-friendly product may be used alone. Ammonia oxidizing microorganism composition preparations for use in conjunction with a biome-friendly product may be formulated for cosmetic or therapeutic use.

Biome-friendly or biome-compatible products may be used in conjunction with an ammonia oxidizing microorganism preparation formulated for any mode of delivery, e.g., formulated for targeted delivery to a subject, e.g., to a target tissue, region, system, or organ of a subject. For example, the ammonia oxidizing microorganism preparation to be used in conjunction with a biome-friendly product may be formulated for delivery to the eye, ear, nose, urogenital system, respiratory system, or gastrointestinal system of the subject. The ammonia oxidizing microorganism composition for use with a biome-friendly product may be formulated for targeted delivery based on a condition or disorder of a subject. For instance, the formulation for targeted delivery may be based on a desired local or systemic effect to be achieved, e.g., a local or systemic therapeutic or cosmetic effect.

Biome-friendly cosmetic products that may be used with the present disclosure may be, or include, or be disposed in any one or more of a baby product, *e.g.,* a baby shampoo, a baby lotion, a baby oil, a baby powder, a baby cream; a bath preparation, *e*.*g*., a bath oil, a tablet, a salt, a bubble bath, a bath capsule; an eye makeup preparation, *e*.*g*., an eyebrow pencil, an eyeliner, an eye shadow, an eye lotion, an eye makeup remover, a mascara; a fragrance preparation, *e*.*g*., a colognes, a toilet water, a perfume, a powder (dusting and talcum), a sachet; hair preparations, *e*.*g*., hair conditioners, hair sprays, hair straighteners, permanent waves, rinses, shampoos, tonics, dressings, hair grooming aids, wave sets; hair coloring preparations, *e*.*g*., hair dyes and colors, hair tints, coloring hair rinses, coloring hair shampoos, hair lighteners with color, hair bleaches; makeup preparations, *e*.*g*., face powders, foundations, leg and body paints, lipstick, makeup bases, rouges, makeup fixatives; manicuring preparations, *e*.*g*., basecoats and undercoats, cuticle softeners, nail creams and lotions, nail extenders, nail polish and enamel, nail polish and enamel removers; oral hygiene products, *e*.*g*., dentrifices, mouthwashes and breath fresheners; bath soaps, *e*.*g*., foaming body cleansers, and detergents, deodorants, douches, feminine hygiene deodorants; shaving preparations, *e*.*g*., aftershave lotions, beard softeners, talcum, preshave lotions, shaving cream, shaving soap; skin care preparations, *e*.*g*., cleansing, depilatories, face and neck, body and hand, foot powders and sprays, moisturizing, night preparations, paste masks, skin fresheners; and suntan preparations, *e*.*g*., gels, creams, and liquids, and indoor tanning preparations.

Products, e.g., microbiome-compatible cosmetic products, e.g., shampoos, conditioners, and cleansers, as described herein may be used in conjunction with the treatment of a condition, disease, or disorder. These cosmetic products may be used in conjunction with administration of the ammonia oxidizing microorganisms for therapeutic or cosmetic purposes. For example, throughout the treatment period or cosmetic period of time of administering the ammonia oxidizing bacteria to a subject, the microbiome-compatible cosmetic products may be used. The microbiome-compatible cosmetic products may be used for a period of time prior to commencement of treatment of the therapeutic or cosmetic condition through administration of ammonia oxidizing bacteria to a subject. The microbiome-compatible cosmetic products may be used for a period of time subsequent to commencement of treatment of the therapeutic or cosmetic condition through administration of ammonia oxidizing bacteria to a subject. The microbiome-compatible cosmetic products may be used for a period of time subsequent to discontinuation of therapeutic or cosmetic treatment of the condition through administration of ammonia oxidizing bacteria to a subject.

The subject may apply one or more cosmetic product, and wait a period of time before administration of the ammonia oxidizing microorganisms. The subject may administer the ammonia oxidizing microorganisms, and wait a period of time before applying one or more cosmetic products.

The period of time the subject may wait may be about 1 minute, 5 minutes, 10, 15, 20, 25, 30, 45, 60, 90, 120 minutes, or 3 hours, 4, 5, 6, 7, 8, 12, 18, 24 hours after applying one or more cosmetic product and prior to administration of ammonia oxidizing microorganisms.

The period of time the subject may wait may be about 1 minute, 5 minutes, 10, 15, 20, 25, 30, 45, 60, 90, 120 minutes, or 3 hours, 4, 5, 6, 7, 8, 12, 18, 24 hours after administering the ammonia oxidizing microorganisms and prior to applying one or more cosmetic products.

### Prophetic Example

A prospective, controlled, double blind, single center, randomized, 3 arm study will be conducted to assess the efficacy of AOM delivered as an intranasal spray to subjects with asthmatic allergic rhinitis and allergic rhinosinusitis. A dose of 140 µL per nostril (1x109 cells/mL) will be administered twice-a-day for 7 days to a first group. A dose of 140 µL per nostril (4x109 cells/mL) will be administered twice-a-day for 7 days to a second group. The third group will receive vehicle (140 µL per nostril) twice a day for 7 days. The study will be driven by the pro-inflammatory effects of ragweed particles challenge in a human model of airways inflammation.

The study is expected to demonstrate that AOM inhibits airway inflammation driven by ragweed challenge alone, as demonstrated by changes in cytokine concentration in nasal fluid. The effect is expected to be greater in the higher concentration population in comparison to both the lower concentration and vehicle populations.

## Claims

1. A preparation comprising ammonia oxidizing microorganisms (AOM) for use in a method of treating allergic rhinitis in a subject, the method comprising:
administering an effective amount of the preparation comprising AOM to the nasal cavity of the subject.

2. The preparation for use according to claim 1, wherein intranasal administration comprises topical application, inhalation, instillation, or olfactory transfer administration.

3. The preparation for use according to claim 1, wherein:
(i) the nasal cavity of the subject is substantially cleared when the preparation is administered; or
(ii) the preparation is to be administered subsequent to administration of an antibiotic or a nasal cavity cleansing preparation.

4. The preparation for use according to claim 1, wherein:
(i) the method further comprises administering a second amount of the preparation to the subject;
(ii) the therapeutically effective dose of AOM is about or greater than about 1 x 10³, 10⁴, 10⁵, 10⁶, 10⁷, 10⁸, 10⁹, 10¹⁰, 10¹¹, 10¹², 10¹³, or 10¹⁴ CFU;
(iii) the preparation comprises AOM in a buffer solution, e.g., an aqueous buffer solution, optionally comprising disodium phosphate and magnesium chloride, for example, 50 mM Na₂HPO₄ and 2 mM MgCl₂ in water; or
(iv) a biome-friendly product is used in connection with the preparation comprising AOM.

5. The preparation for use according to claim 1, wherein the preparation:
(i) is formulated to be compatible with the mucous membrane of the nasal cavity of the subject;
(ii) is formulated for immediate release or extended release;
(iii) is to be administered as part of a combination therapy; or
(iv) is substantially free of other organisms; or
(v) comprises between about 1 x 10³ CFU/mL to about 1 x 10¹⁴ CFU/mL AOM.

6. The preparation for use according to claim 1, wherein the AOM:
(i) comprise ammonia oxidizing bacteria (AOB);
(ii) comprise *Nitrosomonas, Nitrosococcus, Nitrosospira, Nitrosocystis, Nitrosolobus, Nitrosovibrio,* and combinations thereof;
(iii) is *Nitrosomonas eutropha* (*N. eutropha*);
(iv) is *N. eutropha* D23, having ATCC accession number PTA-121157; or
(v) are capable of converting ammonia or ammonium to nitrite at a rate of at least about 1 pmol/min/mg protein, e.g., at least about 0.1 nmol/min/mg protein.

7. The preparation for use according to claim 1, wherein the preparation:
(i) is a nasal drop, spray, aerosol, or mist;
(ii) is formulated for treatment of a neurological disorder in a subject;
(iii) is formulated for treatment of a nasal or sinus disorder in a subject; or
(iv) comprises AOM and other organisms, e.g., a community of organisms.

## Patentansprüche

1. Eine Zubereitung, die ammoniakoxidierende Mikroorganismen (AOM) umfasst, zur Verwendung in einem Verfahren zur Behandlung einer allergischen Rhinitis bei einem Individuum, wobei das Verfahren Folgendes umfasst:
Verabreichen einer wirksamen Menge der Zubereitung, die AOM umfasst, in die Nasenhöhle des Individuums.

2. Zubereitung zur Verwendung nach Anspruch 1, wobei die intranasale Verabreichung topische Anwendung, Inhalation, Instillation oder Verabreichung mittels olfaktorischem Transfer umfasst.

3. Zubereitung zur Verwendung nach Anspruch 1, wobei:
(i) die Nasenhöhle des Individuums im Wesentlichen gereinigt ist, wenn die Zubereitung verabreicht wird; oder
(ii) die Zubereitung zu verabreichen ist, nachdem ein Antibiotikum oder eine Zubereitung zur Reinigung der Nasenhöhle verabreicht worden ist.

4. Zubereitung zur Verwendung nach Anspruch 1, wobei:
(i) das Verfahren ferner das Verabreichen einer zweiten Menge der Zubereitung an das Individuum umfasst;
(ii) die therapeutisch wirksame Dosis von AOM etwa oder mehr als etwa 1 x 10³, 10⁴, 10⁵, 10⁶, 10⁷, 10⁸, 10⁹, 10¹⁰, 10¹¹, 10¹², 10¹³ oder 10¹⁴ CFU beträgt;
(iii) die Zubereitung AOM in einer Pufferlösung umfasst, z. B. einer wässrigen Pufferlösung, die optional Dinatriumphosphat und Magnesiumchlorid umfasst, zum Beispiel 50 mM Na₂HPO₄ und 2 mM MgCl₂ in Wasser; oder
(iv) ein biomfreundliches Produkt in Verbindung mit der Zubereitung, die AOM umfasst, verwendet wird.

5. Zubereitung zur Verwendung nach Anspruch 1, wobei die Zubereitung:
(i) so formuliert ist, dass sie kompatibel mit der Schleimhaut der Nasenhöhle des Individuums ist;
(ii) zur sofortigen Freisetzung oder protrahierten Freisetzung formuliert ist;
(iii) als Teil einer Kombinationstherapie zu verabreichen ist; oder
(iv) im Wesentlichen frei von anderen Organismen ist; oder
(v) zwischen etwa 1 x 10³ CFU/ml bis etwa 1 x 10¹⁴ CFU/ml AOM umfasst.

6. Zubereitung zur Verwendung nach Anspruch 1, wobei die AOM:
(i) ammoniakoxidierende Bakterien (AOB) umfassen;
(ii) *Nitrosomonas, Nitrosococcus, Nitrosospira, Nitrosocystis, Nitrosolobus, Nitrosovibrio* und Kombinationen davon umfassen;
(iii) *Nitrosomonas eutropha* (*N. eutropha*) sind;
(iv) *N. eutropha* D23 mit der ATCC-Zugangsnummer PTA-121157 sind; oder
(v) in der Lage sind, Ammoniak oder Ammonium mit einer Rate von mindestens etwa 1 pmol/min/mg Protein, z. B. mindestens etwa 0,1 nmol/min/mg Protein, in Nitrit umzuwandeln.

7. Zubereitung zur Verwendung nach Anspruch 1, wobei die Zubereitung:
(i) ein Nasentropfen, Spray, Aerosol oder Nebel ist;
(ii) zur Behandlung einer neurologischen Störung bei einem Individuum formuliert ist;
(iii) zur Behandlung einer Nasen- oder Nebenhöhlenstörung bei einem Individuum formuliert ist; oder
(iv) AOM und andere Organismen, z. B. eine Gemeinschaft von Organismen umfasst.

## Revendications

1. Préparation comprenant des micro-organismes oxydant l'ammoniac (MOA) destinée à être utilisée dans un procédé de traitement de la rhinite allergique chez un sujet, le procédé comprenant :
l'administration d'une quantité efficace de la préparation comprenant des MOA dans la cavité nasale du sujet.

2. Préparation destinée à être utilisée selon la revendication 1, dans laquelle l'administration intranasale comprend l'application topique, l'inhalation, l'instillation, ou l'administration par transfert olfactif.

3. Préparation destinée à être utilisée selon la revendication 1, dans laquelle :
(i) la cavité nasale du sujet est sensiblement dégagée lorsque la préparation est administrée ; ou
(ii) la préparation doit être administrée après l'administration d'un antibiotique ou d'une préparation de nettoyage des fosses nasales.

4. Préparation destinée à être utilisée selon la revendication 1, dans laquelle :
(i) le procédé comprend en outre l'administration d'une deuxième quantité de la préparation au sujet ;
(ii) la dose thérapeutiquement efficace de MOA est environ ou supérieure à environ 1 x 10³, 10⁴, 10⁵, 10⁶, 10⁷, 10⁸, 10⁹, 10¹⁰, 10¹¹, 10¹², 10¹³, ou 10¹⁴ UFC ;
(iii) la préparation comprend des MOA dans une solution tampon, par ex. une solution tampon aqueuse, comprenant facultativement du phosphate disodique et du chlorure de magnésium, par exemple 50 mM de Na₂HPO₄ et 2 mM de MgCl₂ dans de l'eau ; ou
(iv) un produit respectueux du biome est utilisé en relation avec la préparation comprenant des MOA.

5. Préparation destinée à être utilisée selon la revendication 1, dans laquelle la préparation :
(i) est formulée pour être compatible avec la membrane muqueuse de la cavité nasale du sujet ;
(ii) est formulée pour une libération immédiate ou prolongée ;
(iii) doit être administrée dans le cadre d'une thérapie combinée ; ou
(iv) est sensiblement exempte d'autres organismes ; ou
(v) comprend entre environ 1 x 10³ UFC/mL à environ 1 x 10¹⁴ UFC/mL de MOA.

6. Préparation destinée à être utilisée selon la revendication 1, dans laquelle les MOA :
(i) comprennent des bactéries oxydant l'ammoniac (BOA) ;
(ii) comprennent *Nitrosomonas, Nitrosococcus, Nitrosospira, Nitrosocystis, Nitrosolobus, Nitrosovibrio,* et des combinaisons de ceux-là ;
(iii) est *Nitrosomonas eutropha* (*N. eutropha*) ;
(iv) est *N. eutropha* D23, portant le numéro d'accès ATCC PTA-121157 ; ou
(v) sont capables de convertir l'ammoniac ou l'ammonium en nitrite à un taux d'au moins environ 1 pmol/min/mg de protéine, par ex. au moins 0,1 nmol/min/mg de protéine.

7. Préparation destinée à être utilisée selon la revendication 1, dans laquelle la préparation :
(i) est une goutte nasale, un spray, un aérosol, ou un brouillard ;
(ii) est formulée pour le traitement d'un trouble neurologique chez un sujet ;
(iii) est formulée pour le traitement d'un trouble nasal ou sinusal chez un sujet ; ou
(iv) comprend des MOA et d'autres organismes, par ex. une communauté d'organismes.
